# EUROPÄISCHE PATENTSCHRIFT

(11) **EP 1 004 668 B1**
(45) Veröffentlichungstag und Bekanntmachung des Hinweises auf die Patenterteilung: **05.01.2005**
(21) Anmeldenummer: 99122909.7
(22) Anmeldetag: 18.11.1999
(51) Int. Cl.: C12N 15/12, C12N 15/11, C12N 15/85, C12N 5/10, C07K 14/47, C07K 16/18, A61K 38/17, A61K 39/395, G01N 33/53

(54) **Regulatorisches Protein pKe 83 aus humanen Keratinozyten**
Regulatory protein pKe 83 derived from human keratinocytes
Protéine régulatrice pKe 83 de keratinocytes humains

(30) Priorität: 26.11.1998 DE 19854672; 07.12.1998 DE 19856301
(43) Veröffentlichungstag der Anmeldung: 31.05.2000
(73) Patentinhaber: Kramer, Michael, 64319 Pfungstadt (DE)
(72) Erfinder: Kramer, M. D. PD Dr. med., 64319 Pfungstadt (DE); Bechtel, Michael, Dr., 69198 Schriesheim (DE); Reinartz, Jeanette, Dr. rer. nat., 69121 Heidelberg (DE); Schäfer, Birgit, Dr. med., 69124 Heidelberg-Kirchheim (DE); Wallich, Reinhard, PD Dr. rer.nat., 69118 Heidelberg (DE)
(74) Vertreter: Rudolph, Ulrike, Dr.

(56) Entgegenhaltungen:
- EMBL DATABASE; EMHUM1.AB020710; ACCESSION-NO.: AB020710, 9. Februar 1999 (1999-02-09), XP002140479 & NAGASE, T. ET AL.: "Prediction of the coding sequences of unidentified human genes. XII. The complete sequences of 100 new cDNA clones from brain which code for large proteins in vitro" DNA RESEARCH, Bd. 5, Dezember 1998 (1998-12), Seiten 355-364, XP002111974
- JAEGER, C.: "Identification of a novel transmembrane-protein (pKe#192/MAP17) expressed by human keratinocytes in the upper stratum granulosum of the epidermis" JOURNAL OF INVESTIGATIVE DERMATOLOGY, Bd. 112, Nr. 4, April 1999 (1999-04), Seite 574 XP000909987
- HILLIER, L. ET AL.: "WashU-NCI human EST Project" EMBL DATABASE; EMEST7.AI205014; ACCESSION-NO.: AI205014, 19. Oktober 1998 (1998-10-19), XP002140480
- HILLIER, L. ET AL.: "WashU-Merck EST Project 1997" EMBL DATABASE; EMEST28.HS1214076; ACCESSION-NO.: AA418645, 14. Mai 1997 (1997-05-14), XP002140481
- KOMINE, M. ET AL.: "The activated keratinocytes" ACTA DERMATOVENEROLOGICA A.P.A., Bd. 4, Nr. 4, 1995, Seiten 169-173, XP000915531
- JIANG, C.-K., ET AL.: "Epidermal growth factor and transforming growth factor alpha specifically induce the activation- and hyperproliferation-associated keratins 6 and 16" PROC.NATL.ACAD.SCI.USA, Bd. 90, Nr. 14, 1993, Seiten 6786-6790, XP000910179
- KNAPP, B. ET AL.: "Three cDNA sequences of mouse type I keratins" JOURNAL OF BIOLOGICAL CHEMISTRY, Bd. 262, Nr. 2, 1987, Seiten 938-945, XP000910011

## Beschreibung

Die Erfindung betrifft ein isoliertes Polypeptid, das einem natürlicherweise in Keratinozyten vorkommenden und im aktivierten Zustand der Keratinozyten verstärkt exprimierten Protein gleicht oder ähnlich, d.h. in Funktion und Wirkung gleich ist. Sie betrifft außerdem eine isolierte Nukleinsäure, die ein solches für humane Keratinozyten typisches Polypeptid bzw. Protein kodiert, sowie die Verwendung dieses Polypeptids und dieser Nukleinsäure für nachweisende, insbesondere diagnostische, und/oder für therapeutische Zwecke, und Reagenzien, die unter Verwendung wenigstens eines dieser Moleküle hergestellt sind, insbesondere rekombinante Vektormoleküle und Antikörper.

Nach dem gegenwärtigen Stand der Technik werden in der Dermatotherapie zur Beeinflussung epidermaler Störungen wie z.B. der Autoimmundermatosen "Pemphigus vulgaris" und "Bullöses Pemphigoid" im wesentlichen Medikamente mit breitem Wirkungsspektrum eingesetzt, insbesondere lokal bzw. systemisch applizierte Glukokortikoide, Vitamin-A-Säure-Derivate, Antimetabolite und Zytostatika, oder es wird mit mehr oder weniger unspezifischen Maßnahmen, wie z.B. der sog. "Farbstofftherapie" oder der "Lichttherapie", behandelt. Die bekannten Wirkstoffe bzw. Maßnahmen haben jedoch allesamt den Nachteil, daß sie wenig spezifisch sind und damit naturgemäß zahlreiche Nebenwirkungen hervorrufen.

Die Bereitstellung spezifischerer Wirkstoffe scheiterte bislang an dem in der Dermatologie seit langem bestehenden grundsätzlichen Problem, daß die Zahl der zellulären Zielmoleküle, im folgenden allgemein als Zielstrukturen ("Targets") benannt, die als Angriffspunkt für eine (spezifische) Beeinflussung des zellulären Stoffwechsels - insbesondere unter medizinischen oder auch kosmetischen Gesichtspunkten - dienen könnten, in epidermalen Keratinozyten eng begrenzt ist.

Aufgabe der vorliegenden Erfindung ist es deshalb, neue Zielstrukturen in epidermalen Keratinozyten bereitzustellen, die als Angriffspunkt für Diagnostika, Therapeutika, Kosmetika oder allgemein für die Beeinflussung des zellulären Stoffwechsels dienen können.

Eine Lösung dieser Aufgabe besteht in der Bereitstellung eines Polypeptids bzw. Proteins der eingangs genannten Art, das bei aktivierten Keratinozyten aufreguliert, d.h. vermehrt exprimiert bzw. produziert und auf einem höheren Konzentrationsspiegel gehalten wird, und das die im Sequenzprotokoll **SEQ ID NO: 3** oder **SEQ ID NO: 4** oder **SEQ ID NO: 6** dargestellte Aminosäuresequenz oder ein durch Aminosäuresubstitution, -deletion, - insertion oder -inversion daraus entstandenes Allel oder Derivat dieser Aminosäuresequenz aufweist. Das Polypeptid mit der Aminosäuresequenz gemäß **SEQ ID NO: 3** oder **SEQ ID NO: 4** oder **SEQ ID NO: 6** wird im folgenden auch mit Protein "pKe#83" bezeichnet.

Eine weitere Lösung der genannten Aufgabe besteht in der Bereitstellung einer isolierten Nukleinsäure, die ein Protein kodiert, das einem natürlicherweise in humanen Keratinozyten vorkommenden und im aktivierten Zustand der Keratinozyten verstärkt exprimierten Protein gleicht oder ähnlich ist, und die im Sequenzprotokoll **SEQ ID NO: 1** dargestellte Nukleotidsequenz oder eine hierzu komplementäre Nukleotidsequenz aufweist, wobei in diesem Sequenzprotokoll **SEQ ID NO: 1** anstelle von "T" auch "U" stehen kann. Zu dieser erfindungsgemäßen Gruppe von Nukleinsäuren bzw. Nukleotidsequenzen gehören insbesondere auch Splice-Varianten (z.B. **SEQ ID NO: 2** oder **SEQ ID NO: 5**) und Sense- oder Antisense-Oligonukleotide, die mit der im Sequenzprotokoll **SEQ ID NO: 1** oder mit der im Sequenzprotokoll **SEQ ID NO: 7** dargestellten Nukleotidsequenz hybridisieren, vorzugsweise identisch mit bzw. (partiell) komplementär zu dieser sind. Zwei bevorzugte Splice-Varianten der erfindungsgemäßen Nukleotidsequenz gemäß **SEQ ID NO: 1** sind in den Sequenzprotokollen **SEQ ID NO: 2** und **SEQ ID NO: 5** dargestellt.

Die Erfindung umfaßt infolgedessen auch Proteine bzw. Polypeptide der eingangs genannten Art, die eine Aminosäuresequenz aufweisen, welche aus einer solchen Splice-Variante resultiert, insbesondere aus der Splice-Variante einer mRNA, die mit der im Sequenzprotokoll **SEQ ID NO: 1** angegebenen Nukleotidsequenz identisch oder ganz oder teilweise komplementär dazu ist.

Die erfindungsgemäßen Sense- und Antisense-Oligonukleotide umfassen jeweils mindestens 6, vorzugsweise 8 - 25 Nukleotide.

Der Begriff "hybridisiert" bezieht sich auf die im Stand der Technik bekannten Hybridisierungsverfahren unter üblichen, insbesondere unter hoch stringenten Hybridisierungsbedingungen. Die konkreten Hybridisierungsparameter wählt der Fachmann anhand der eingesetzten Nukleotidsequenz und seines allgemeinen Fachwissens (vgl.: *Current Protocols in Molecular Biology, Vol.* 1, *1997, John Wiley & Sons Inc., Suppl. 37, Chapter 4.9.14*).

Neben den in den Sequenzprotokollen **SEQ ID NO: 1, SEQ ID NO: 2** und **SEQ ID NO: 5** gezeigten Nukleotidsequenzen und den diesen Sequenzen im Rahmen der Degeneration des genetischen Codes entsprechenden Nukleotidsequenzen umfaßt die vorliegende Erfindung auch solche Nukleotidsequenzen, die damit unter stringenten Bedingungen hybridisieren. Der Begriff "hybridisieren" bzw. "Hybridisierung" gemäß vorliegender Erfindung wird wie bei *Sambrook et al., Molecular Cloning, A Laboratory Manual, Cold Spring Harbor, Laboratory, Press, 1989, 1.101 bis 1.104* verwendet. Demnach spricht man von einer Hybridisierung unter stringenten Bedingungen, wenn nach Waschen für eine Stunde mit 1 x SSC und 0,1% SDS, vorzugsweise mit niedriger konzentriertem SSC, insbesondere 0,2 x SSC, bei einer Temperatur von wenigstens 55°C, vorzugsweise 62°C und besonders bevorzugt 68°C noch ein positives Hybridisierungssignal beobachtet wird. Jede Nukleotidsequenz, die unter derartigen Waschbedingungen mit einer Nukleotidsequenz gemäß **SEQ ID NO: 1, SEQ ID NO: 2** oder **SEQ ID NO: 5** oder mit einer der Sequenz gemäß **SEQ ID NO: 1** oder **SEQ ID NO: 2** oder **SEQ ID NO: 5** im Rahmen der Degeneration des genetischen Codes entsprechenden Nukleotidsequenz hybridisiert, gehört zum Gegenstand der vorliegenden Erfindung.

Die erfindungsgemäße(n) Nukleinsäure(n) kann (können) sowohl aus einer natürlichen Quelle als auch synthetisch oder halbsynthetisch gewonnen werden. In der Praxis hat sich besonders die Ausführung als cDNA bewährt.

Das Polypeptid, das die Aminosäuresequenz gemäß **SEQ ID NO: 3** aufweist und von der im Sequenzprotokoll **SEQ ID NO: 1** dargestellten Nukleinsäure kodiert wird, und das im folgenden als Protein "pKe#83" bezeichnet ist, wird in humanen epidermalen Keratinozyten aufreguliert, nämlich verstärkt exprimiert (produziert) und auf einem im Vergleich zum Ausgangszustand signifikant höheren Konzentrationsspiegel gehalten, wenn sich diese Zellen im "aktivierten" Zustand befinden, d.h. unter anderem im Zustand der Proliferation und/oder Migration, z.B. nach einer unfallbedingten Hautverletzung oder bei den autoimmunologisch ausgelösten bullösen Dermatosen "Pemphigus vulgaris" (ausgelöst durch Autoantikörper gegen Desmosomen) und "Bullöses Pemphigoid" (ausgelöst durch Autoantikörper gegen Hemidesmosomen). Der aktivierte Zustand der humanen epidermalen Keratinozyten äußert sich auch in einer im Vergleich zum Ruhezustand (Ausgangszustand) erhöhten Expression der bekannten Aktivierungsmarker uPA (Urokinase-Typ Plasminogenaktivator) und uPA-R (Rezeptor für Urokinase-Typ Plasminogenaktivator) und kann anhand dieser Marker qualitativ und quantitativ nachgewiesen werden (vgl.: *Schäfer, et al., 1996: Dispase-mediated basal detachment of cultured keratinocytes induces urokinase-type plasminogen activator (uPA) and its receptor (uPA-R, CD87), Exp. Cell Res. 228, pp. 246-253*).

Das Protein pKe#83 weist eine sog. Prenyl-Gruppen-Bindungsstelle ("CAAX Box") auf. Hierbei handelt es sich um eine Bindungsstelle, die bei einer Vielzahl eukaryontischer Proteine eine posttranslationelle Veränderung erlaubt, indem eine Farnesyl- oder eine Gerany-Geranyl-Gruppe an einen Cysteinrest angehängt wird, der drei Aminosäuren vom C-Terminus entfernt ist, und wobei die beiden am C-Terminus gelegenen Aminosäuren in der Regel aliphatisch sind. Ras Proteine und eine Vielzahl von G-Proteinen weisen eine solche CAAX Box auf.

Zudem besitzt das Protein "pKe#83" eine Reihe putativer Phosphorylierungsstellen. Die genannten Motive weisen darauf hin, daß das Protein pKe#83 in Signaltransduktionsabläufe eingebunden ist.

Mit der (isolierten) Bereitstellung des Proteins "pKe#83", nämlich mit der Beschreibung von Nukleotidsequenzen, die dieses Protein kodieren, und mit der Angabe (einer) seiner Aminosäuresequenz(en) ist es möglich, den Stoffwechsel physiologisch aktiver bzw. aktivierter Keratinozyten - und selbstverständlich auch anderer das Protein "pKe#83" exprimierender Zellen - gezielt zu beeinflussen, insbesondere zu Zwecken der medizinischen Therapie und kosmetischen Behandlung.

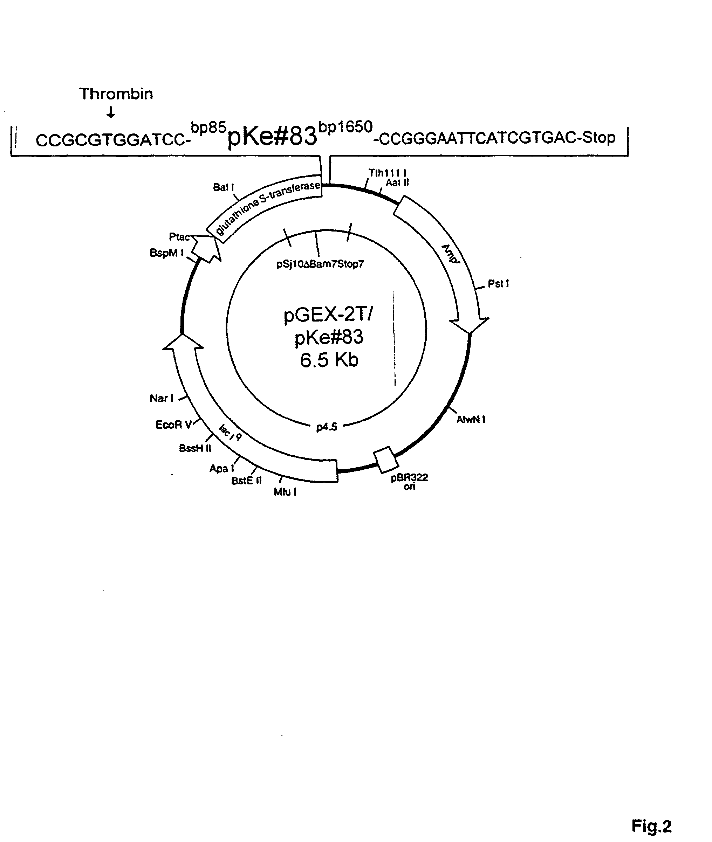

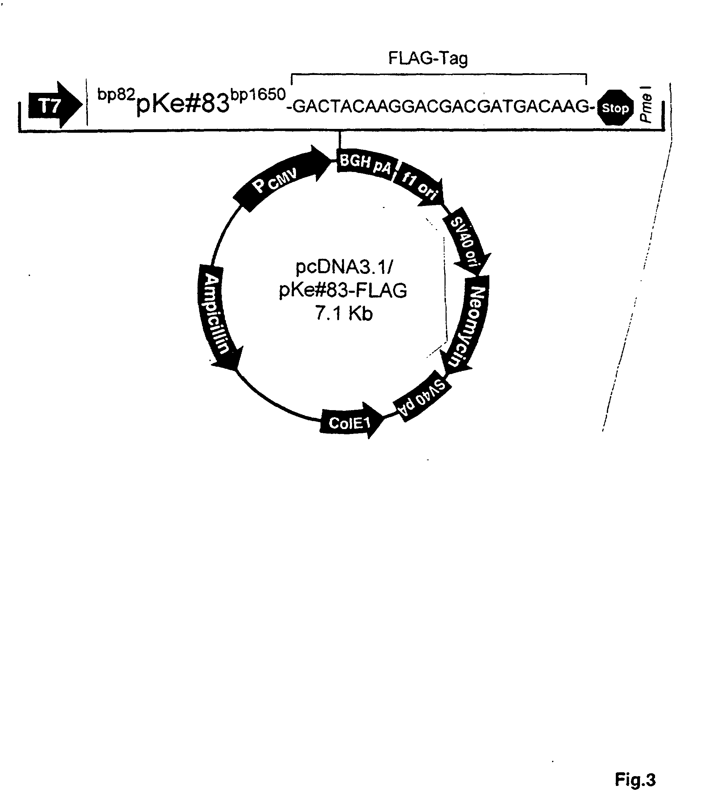

Die Erfindung betrifft desweiteren rekombinante DNS-Vektormoleküle, die eine erfindungsgemäße Nukleinsäure umfassen, und die die Fähigkeit zur Expression eines in humanen Keratinozyten vorkommenden und im aktivierten Zustand der Keratinozyten verstärkt exprimierten Proteins, insbesondere des Proteins pKe#83, in einer prokaryontischen oder eukaryontischen Zelle aufweisen. Bei diesen DNS-Vektormolekülen handelt es sich vorzugsweise um Abkömmlinge des Plasmids pUEX-1 und/oder des Plasmids pGEX-2T und/oder des Plasmids pcDNA3.1, da sich diese Vektoren in der Praxis als sehr gut geeignet erwiesen haben. Besonders bevorzugt sind das Vektorkonstrukt pGEX-2T/pKe#83 gemäß dem in Fig. 2 offenbarten Vektorprotokoll und das Vektorkonstrukt pcDNA3.1/pKe#83-FLAG gemäß dem in Fig. 3 offenbarten Vektorprotokoll. Als eukaryontische Zelle kommen insbesondere Zellen aus Zellkulturen, z.B. Cos-Zellen, in Betracht, ebensogut kann die betreffende Zelle aber auch Bestandteil eines lebenden Organismus, z. B. einer transgenen Maus, sein.

Die Erfindung umfaßt deshalb auch transformierte Wirtszellen, die eine erfindungsgemäße Nukleinsäure enthalten, die mit einem aktivierbaren Promotor gekoppelt ist, der in diesen Zellen natürlicherweise oder als Folge einer Rekombination enthalten ist, und die (infolgedessen) die Fähigkeit zur Expression eines in humanen Keratinozyten vorkommenden und im aktivierten Zustand der Keratinozyten verstärkt exprimierten Proteins, insbesondere des Proteins "pKe#83", besitzen.

Die Erfindung betrifft ferner die Verwendung einer erfindungsgemäßen Nukleinsäure oder eines erfindungsgemäßen Vektormoleküls zur Herstellung transgener Säugetiere, insbesondere Mäuse oder Ratten.

Die erfindungsgemäßen Transfektanten ermöglichen Forschungs- und Entwicklungsarbeiten zum Zweck der weitergehenden Aufklärung der durch das Protein "pKe#83" induzierten Veränderungen der Zellmorphologie und zellulären Basisfunktionen wie Proliferation, Adhäsion, Migration und Differenzierung, insbesondere im Hinblick auf die Beantwortung der Frage, ob das Protein "pKe#83" selbst eine "pathogene" Aktivität besitzt.

Gegenstand der vorliegenden Erfindung ist außerdem ein Reagenz zum indirekten Nachweis eines in humanen Keratinozyten vorkommenden und im aktivierten Zustand der Keratinozyten verstärkt exprimierten Proteins, insbesondere des Proteins "pKe#83", wobei dieses Reagenz dadurch charakterisiert ist, daß es wenigstens eine erfindungsgemäße Nukleinsäure umfaßt. "Zum indirekten Nachweis" bedeutet in diesem Zusammenhang, daß tatsächlich die das Protein kodierende mRNA direkt nachgewiesen wird - und somit das Protein nur indirekt (vermittels dieser mRNA).

Das Protein "pKe#83" und die damit, d.h. mit der im Sequenzprotokoll **SEQ ID NO: 3** dargestellten Aminosäuresequenz verwandten Polypeptide, d.h. die Polypeptide, die durch Substitution, Deletion, Insertion und/oder Inversion von der Aminosäuresequenz gemäß **SEQ ID NO: 3** ableitbar sind oder die eine Aminosäuresequenz aufweisen, die aus einer Splice-Variante einer mRNA resultiert, welche mit der Nukleotidsequenz gemäß Sequenzprotokoll **SEQ ID NO: 1** oder einer Teilsequenz davon identisch oder komplementär dazu ist oder zumindest hybridisiert, bieten vielfältige Anwendungsmöglichkeiten auf dem Gebiet der dermatologischen Forschung und Entwicklung. Insbesondere können Antikörper gegen diese Polypeptide bzw. Proteine hergestellt werden, die dann mit entsprechender Modifikation entweder als Diagnostika oder als Therapeutika oder auch als Kosmetika ("cosmeceuticals") einsetzbar sind.

Die Erfindung umfaßt folglich auch die Verwendung eines solchen Proteins bzw. Polypeptids zur Herstellung eines (monoklonalen oder polyklonalen) Antikörpers gegen dieses Polypeptid, den besagten Antikörper selbst und ebenso seine Verwendung zur diagnostischen und/oder therapeutischen Behandlung von dermatologischen Erkrankungen, zur kosmetischen Behandlung der Epidermis und zur Diagnostik und/oder kosmetischen Behandlung von anderen das Protein "pKe#83" exprimierenden Geweben oder Organen.

Auch Sense- und/oder Antisense-Oligonukleotide kommen nach neueren wissenschaftlichen Erkenntnissen als Wirkstoffe für eine Pharmakotherapie in Betracht (vgl. G. Hartmann et al. 1998: *Antisense-Oligonukleotide*, Deutsches Ärzteblatt 95, Heft 24, C1115-C1119) - und überdies als Wirkstoffe mit einem in der Pharmakotherapie grundsätzlich neuen Wirkprinzip.

Die vorliegende Erfindung betrifft deshalb auch die Verwendung erfindungsgemäßer Sense- oder Antisense-Oligonukleotide zur Diagnostik und/oder therapeutischen Behandlung, insbesondere von dermatologischen Erkrankungen, oder zur kosmetischen Behandlung, insbesondere der Epidermis.

Eine technisch und wirtschaftlich bedeutende Einsatzmöglichkeit eines erfindungsgemäßen Polypeptids und ebenso einer erfindungsgemäßen Nukleinsäure besteht nicht zuletzt auch darin, daß mit Hilfe eines solchen Moleküls in einem "Screening"-Verfahren aus einer sehr großen Anzahl bereitstehender Stoffe solche herausselektiert werden können, die spezifisch an die betreffende Nukleinsäure oder das betreffende Polypeptid binden. Diese Stoffe können dann als Ausgangsmaterial (Leitstruktur) für die Entwicklung pharmakologisch einsetzbarer Substanzen dienen und bieten damit die Voraussetzungen für die Entwicklung alternativer Pharmazeutika zur Diagnose und Therapie, insbesondere der eingangs erwähnten dermatologischen Erkrankungen und/oder anderer Erkrankungen, bei denen das Protein "pKe#83" eine wichtige Rolle spielt.

Im Hinblick darauf betrifft die Erfindung auch die Verwendung eines erfindungsgemäßen Polypeptids oder einer erfindungsgemäßen Nukleinsäure zur Identifizierung pharmakologisch einsetzbarer Substanzen, die an das Polypeptid bzw. die Nukleinsäure binden und dadurch dessen bzw. deren Funktion und/oder Expression beeinflussen, insbesondere inhibierend oder aktivierend wirken.

Die Erfindung wird im folgenden anhand von Herstellungs- und Anwendungsbeispielen näher erläutert. Von den im Rahmen dieser Beispiele erwähnten Figuren zeigen
- Fig. 1:: einen rt-PCR-Nachweis von "pKe#83"-spezifischer mRNA
- Fig. 2:: das Vektorkonstrukt pGEX-2T/pKe#83
- Fig. 3:: das Vektorkonstrukt pcDNA3.1/pKe#83-FLAG
- Fig.4:: einen Immunoblot-Nachweis von rekombinantem pKe#83 Protein in E. Coli-Zellen nach Transfektion mit dem Vektorkonstrukt pGEX-2T/pKe#83
- Fig. 5:: einen Immunoblot-Nachweis von rekombinantem pKe#83 Protein in Cos-Zellen nach Transfektion mit dem Vektorkonstrukt pcDNA3.1/pKe#83-FLAG
- Fig. 6:: einen Immunblot-Nachweis von anti-Protein pKe#83-Antikörpern aus Kaninchenserum auf rekombinantem pKe#83-Protein (A) und einen Immunblot-Nachweis von exprimiertem Protein pKe#83 in transfizierten Cos-Zellen mit anti-Protein-pKe#83-Antikörpern aus Kaninchenserum (B)
- Fig. 7:: einen "Sandwich"-ELISA-Test unter Verwendung von Antikörpern, die gegen das Protein pKe#83 gerichtet sind.
- Fig. 8:: einen Immunfluoreszenztest unter Verwendung von Kaninchen "anti-pKe#83 IgG" auf Normalhautschnitten (C), NHEK-Sheets direkt nach Dispase-induzierter Ablösung und (A) und NHEK-Sheets 8 Stunden nach Dispase-induzierter Ablösung (B).
- Fig. 9:: Keratinozyten (HaCaT-Zellen) nach Behandlung mit pKe#83-spezifischen Antisense Oligonukleotiden (B) und Kontroll-Oligonukleotiden (A)

### Beispiel 1: Herstellung des Proteins "pKe#83"

### A) Gewinnung und Herstellung eines Polynukleotids, das das Protein "pKe#83" kodiert

Als Polynukleotidquelle dienten humane epidermale Keratinozyten einer Zellkultur bzw. eines Zellkulturmodells, das in der Publikation von *Schäfer B. M. et al., 1996: Dispase-mediated basal detachment of cultured* *keratinocytes induces urokinase-type plasminogen activator (uPA) and its receptor (uPA-R, CD87), Exp. Cell Res. 228, pp. 246-253*, ausführlich beschrieben ist. Auf den Inhalt dieser Publikation wird hiermit ausdrücklich Bezug genommen. Diese Zellkultur bzw. dieses Zellkulturmodell zeichnet sich dadurch aus, daß sie/es erlaubt, Keratinozyten durch enzymatische Zerstörung der Zell/Matrix-Kontakte, beispielsweise durch eine Dispaseinduzierte Ablösung der Keratinozyten von der Kulturmatrix, vom ruhenden [uPA⁻/uPA-R] in den aktivierten [uPA⁺/uPA-R⁺] Zustand zu überführen. Die Induktion des aktivierten Zustands ist reversibel: die (erneute) Ausbildung eines konfluenten (= maximal dicht gewachsenen), mehrschichtigen Zellverbands aus differenzierten Keratinozyten führt zur Abregulierung von uPA und uPA-R, d.h. zur Drosselung der Produktion und Einstellung auf einem niedrigeren Konzentrationsspiegel (siehe dazu die Publikation von *Schäfer B.M. et al., 1996: Differential expression of urokinase-typ*e *plasminogen activator (uPA), its receptor (uPA-R), and inhibitor type-2 (PAI-2) during differentiation of keratinocytes in an organotypic coculture system. Exp. Cell Res. 220, pp. 415-423*).

Die Zellen dieser Zellkultur bzw. dieses Zellkulturmodells werden im folgenden auch als NHEK (= "normale humane epidermale Keratinozyten") bezeichnet.

Für die Bereitstellung der Zellkultur bzw. des Zellkulturmodells wurden folgende Maßnahmen durchgeführt: Mittels Hautbiopsie erhaltene NHEK wurden über Nacht bei 4°C trypsiniert und anschließend nach der "Feeder Layer"-Technik von *J. G. Rheinwald und H. Green (1975, Serial cultivation of strains of human epidermal keratinocytes: the formation of keratinizing colonies from single cells, Cell 6, pp. 331-334*) in Petrischalen oder in 175 cm² Kulturflaschen für die Dauer von 8 Tagen in Dulbecco's modified Eagle's Medium (DMEM) mit einem Gehalt von 10 Vol.-% fötalem Kälberserum (FCS) und Zusätzen an Adeninhemisulfat, Insulin, Transferrin, Trijodthyronin, Hydrocortison, Forskolin, epidermalem Wachstumsfaktor (EGF) und Antibiotika (Penicillin, Streptomycin und Gentamycin) unter Differenzierungsbedingungen, insbesondere erhöhten Kalziumspiegeln, kultiviert (37°C, 7 % CO₂). Die Kultivierung erfolgte damit gemäß herkömmlicher und im Stand der Technik geläufigen Bedingungen. Unter diesen Bedingungen bilden Keratinozyten konfluente zwei- bis dreischichtige sog. "Epidermisäquivalente" oder Keratinozyten-"Sheets" aus.

Diese Epidermisäquivalente bzw. Keratinozytensheets wurden durch eine 30-minütige Behandlung mit Dispase II (2,4 mg/ml in DMEM ohne FCS) von der Kulturmatrix abgelöst, zweimal in DMEM gewaschen und anschließend für die Dauer von 4 - 8 Stunden in komplettem, konditioniertem DMEM inkubiert. Die Inkubation in konditioniertem DMEM erfolgte, um den Einfluß von frischem FCS auszuschließen. Während der Inkubation fand in diesen flotierenden Keratinozytensheets eine Aufregulierung der bekannten Aktivierungsmarker uPA und uPA-R sowie des hierin erstmals beschriebenen Proteins pKe#83 statt. Die uPA/uPA-R-Aufregulierung war mittels bekannter Techniken wie Enzyme-Linked-Immunosorbent Assay (ELISA), in situ-Hybridisierung und Immunfluoreszenz nachweisbar. Aus den inkubierten Zellen wurde mittels der im Stand der Technik bekannten Guanidinium-Thiocyanat-Phenol-Chloroform-Extraktionsmethode (vgl.: *Chromczynski P. and Sacchi N., 1986: Single-step method of RNA isolation by acid guanidinium thiocyanate-phenol-chloroform extraction. Anal. Biochem. 162: pp. 156-159*) die gesamte RNA gewonnen (Kit "RNA-Clean" der Firma AGS, Heidelberg). Aus der Gesamt-RNA wurde die mRNA mittels Bindung an Poly-T-beschichtete Kügelchen isoliert. Diese mRNA diente als Ausgangsmaterial für den nächstfolgenden Verfahrensschritt der Subtraktionsklonierung.

Für den Einsatz in Kontrollversuchen bzw. für Vergleichspräparate wurde mRNA von adhärenten Keratinozytensheets isoliert, und zwar nach dem gleichen Verfahrensmuster wie vorstehend beschrieben, ausgenommen der Abweichung, daß für die Dauer der Dispasebehandlung zusätzlich zu der Dispase ein Dispasehemmer, z.B. Phosphoramidon (100 µg/ml), appliziert wurde.

Nach dem Prinzip der Subtraktionsklonierung wurde eine Genbank erstellt, die vorzugsweise cDNA der dyshäsionsinduzierten Gene enthielt, d.h. solcher Gene, die nach Ablösung der Keratinozytensheets vermehrt in diesen (bzw. deren Zellen) exprimiert wurden. Zu diesem Zweck wurde die aus den Zellen der adhärenten Keratinozytensheets gewonnene mRNA erneut an poly-T-beschichtete Kügelchen gebunden, auf diesen in einzelsträngige cDNA umgeschrieben und anschließend gegen die mRNA abgelöster, d.h. nicht-adhärenter Keratinozytensheets hybridisiert. Diejenigen mRNA-Moleküle, die lediglich im nicht-adhärenten Zustand, d.h. nach Dyshäsion exprimiert wurden und infolgedessen keinen Hybridisierungspartner fanden, verblieben im Überstand. Sie wurden in cDNA umgeschrieben und in den Klonierungsvektor pUEX-1 kloniert.

Die daraus resultierende Genbank wurde zwecks Überprüfung anschließend noch einem Southernblot-Verfahren mit [³²P]-markierter cDNA adhärenter und nicht-adhärenter Keratinozytensheets unterworfen. Diejenige cDNA oder vielmehr die sie enthaltenden Wirtszellklone - hier des E. coli Stamms MC1061 -, die nach Dyshäsion eine deutliche Aufregulation zeigten, wurden anschließend über Nacht bei 30°C unter üblichen Kulturbedingungen kultiviert bzw. vermehrt. Aus diesen E. coli-Klonen wurde die Plasmid-DNA (pUEX1-cDNA) herauspräpariert, und die aus dem pUEX1-Vektor herausgeschnittenen cDNA-Fragmente wurden mittels Random-priming [³²P]-markiert. Die markierte cDNA wurde als Sonde in Northernblots mit RNA aus adhärenten und nicht-adhärenten Keratinozytensheets eingesetzt. Die Klone, die cDNA enthielten, die bei Verwendung als Sonde im Northernblot-Verfahren kein oder nur ein geringes Signal mit der RNA adhärenter Keratinozyten, dagegen ein deutliches Signal mit RNA nicht-adhärenter Keratinozytensheets erkennen ließen, wurden für den nachfolgenden Verfahrensabschnitt der Sequenzierung ausgewählt.

Bei der Sequenzierung der betreffenden Klone mittels des "nichtradioaktiven Cycle-Sequencing", das eine Modifikation der Sequenzierungsmethode nach Sanger (*F. Sanger et al., 1977: DNA sequencing with chain terminating inhibitors, Proc Natl Acad Sci USA 74: 5463-5467*) darstellt und mittlerweile eine dem Stand der Technik geläufige Methode ist, wurde unter anderem das Gen mit der Nukleotidsequenz gemäß Sequenzprotokoll **SEQ ID NO: 1** und gemäß Sequenzprotokoll **SEQ ID NO: 7** erhalten. Außerdem wurden die in den Protokollen **SEQ ID NO: 2** und **SEQ ID NO: 5** dargestellten Splice-Varianten gefunden. Das Gen mit der Nukleotidsequenz gemäß Protokoll **SEQ ID NO: 1** bzw. **SEQ ID NO: 7** und das zugehörige Protein erhielten die Bezeichnung "pKe#83".

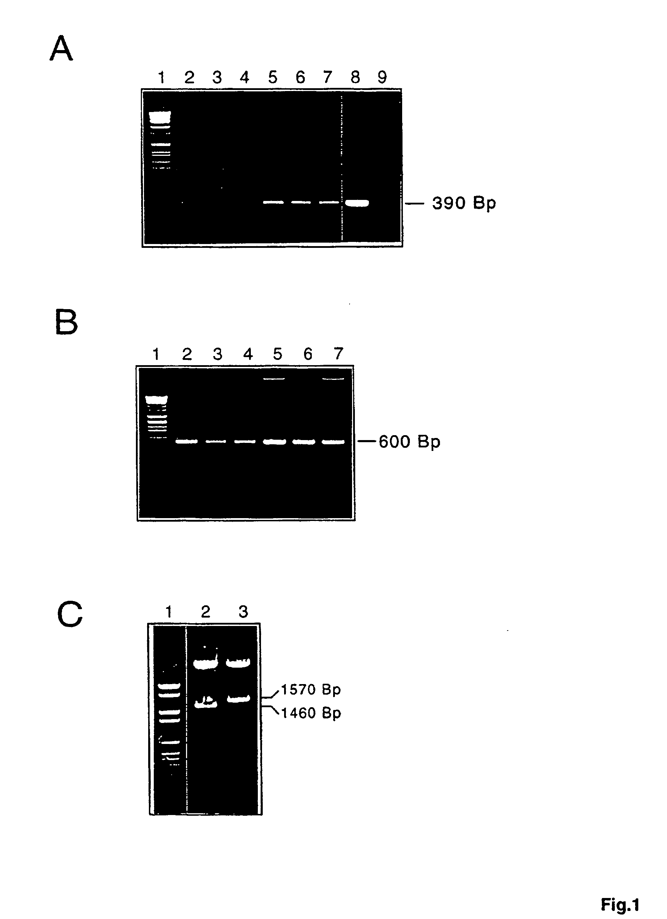

Nähere Untersuchungen der zu dem Gen pKe#83 gehörigen, d.h. pKe#83-spezifischen mRNA (aus abgelösten, d.h. nicht-adhärenten Keratinozytensheets) lieferten die Informationen, daß diese mRNA eine Größe von etwa 2,6 kb (**SEQ ID NO:1**) bis etwa 4,9 kb ( **SEQ ID NO: 7**) aufweist. Die Nukleotidsequenz gemäß **SEQ ID NO:1** bzw. **SEQ ID NO: 7** enthält am 3'Ende an Position 1651-1653 (**SEQ ID NO:1**) bzw. an Position 3895-3897 (**SEQ ID NO: 7**) ein Stopcodon, das den mutmaßlichen Ort des Transkriptionsendes vorgibt. An Position 2612-2617 gemäß **SEQ ID NO:1** bzw. Position 4856-4261 gemäß **SEQ ID NO: 7**, genau 26 Nukleinsäuren vor der poly-A-Site, befindet sich eine sog. Polyadenylation site. Es wurde eine Splice- Variante (**SEQ ID NO: 2**) gefunden, die um 111 Nukleinsäuren (Position 669-780 gemäß **SEQ ID NO:1**) kürzer ist, und eine zweite Splice-Variante (**SEQ ID NO: 5**), die um 108 Nukleinsäuren (Position 670-777 gemäß **SEQ ID NO:1**) kürzer ist. **Fig. 1.C** zeigt das Ergebnis der Klonierung einer pKe#83 -cDNA-Sequenz, es gilt:
- Spur 1 =: DNA Molekulargewichtsmarker VI (154-2176 Bp, Boehringer Mannheim),
- Spur 2 =: SEQ ID NO: 1 (1570 Bp)
- Spur 3 =: SEQ ID NO: 2 (1460 Bp).

Mit Hilfe der Polymerasekettenreaktion konnte gezeigt werden, daß die pKe#83-spezifische mRNA nach Dispase-induzierter Ablösung der NHEK eine Aufregulation erfährt. In **Fig. 1.A** ist das Ergebnis einer Polymerasekettenreaktion nach reverser Transkription (rt-PCR) der mRNA in cDNA und Amplifikation mit pKe#83-spezifischen Oligonukleotid-Primem dargestellt. Dieses Ergebnis beinhaltet die Aussage, daß direkt nach der Ablösung der NHEK nur wenig pKe#83-mRNA vorhanden oder jedenfalls nachweisbar war, daß aber bereits 2 Stunden später eine deutliche Aufregulation festgestellt werden konnte.

### B) Ableitung der Aminosäurenabfolge und Charakterisierung des Proteins "pKe#83" anhand des dafür kodierenden Polynukleotids

Anhand des genetischen Codes der "pKe#83"-cDNA wurde mit Hilfe eines computergestützten Verfahrens (Programm "HUSAR" [= Heidelberg Unix Sequence Analysis Ressources] Version 4.0, Deutsches Krebsforschungszentrum Heidelberg, 1997] von den Nukleotidsequenzen gemäß Sequenzprotokoll **SEQ ID NO: 1** und Sequenzprotokoll **SEQ ID NO: 7** eine Aminosäuresequenz abgeleitet, die in den Sequenzprotokollen **SEQ ID NO: 3** und **SEQ ID NO: 8** dargestellt sind. Die strukturelle Analyse dieser Aminosäuresequenzen gemäß Sequenzprotokoll **SEQ ID NO: 3** und **SEQ ID NO: 8** mit eben diesem Programm lieferte folgende Informationen:

Aus der Aminosäurezusammensetzung des Proteins pKe#83 errechnet sich ein Molekulargewicht von 60380 Da (gemäß **SEQ ID NO: 3**) bzw. 122180 Da (gemäß **SEQ ID NO: 8**) mit einem isoelektrischen Punkt von pH 5,3 (gemäß **SEQ ID NO: 3**) bzw. pH 4,9 (gemäß **SEQ ID NO: 8**).

Das Protein pKe#83 weist eine sog. Prenyl-Gruppen-Bindungsstelle ("CAAX Box") auf und eine Reihe möglicher Phosphorylierungsstellen (9x Proteinkinase C, 15x Caseinkinase II, 2x Tyrosinkinase gemäß **SEQ ID NO: 3** und 24x Proteinkinase C, 29x Caseinkinase II, 5x Tyrosinkinase gemäß **SEQ ID NO: 8**). Diese Motive sind ein Hinweis dafür, daß das Protein pKe#83 in Signaltransduktionsabläufe eingebunden ist. Das Protein pKe#83 gemäß **SEQ ID NO: 8** weist zudem mehrere (acht) Myristylierungsstellen auf.

### Beispiel 2: Nachweis "pKe#83"-spezifischer mRNA in Zellen mittels reverser Polymerasekettenreaktion

Mittels der Polymerasekettenreaktion nach reverser Transkription (rt-PCR) wurde pKe#83-spezifische mRNA in Zellen (NHEK) von Keratinozytensheets nach Dispasebehandlung und in HaCaT-Zellen nachgewiesen. Hierfür wurde RNA aus Zellen von Keratinozytensheets nach Dispasebehandlung und unterschiedlich langer weiterer Inkubationszeit und aus HaCaT-Zellen jeweils mit Standardmethoden (Guanidinium-Thiocyanat-Phenol-Chloroform-Extraktionsmethode) isoliert und nach Standardmethoden in cDNA umgeschrieben. Diese cDNA wurde einer PCR unterzogen, bei der aus der pKe#83-spezifischen cDNA ein Teilfragment von 388 Bp amplifiziert wurde.

Als Primer-Paar wurde eine Kombination aus pKe#83-forward-10 (¹⁰³²GAATAGACCAGAGATGAAAAGGCAG¹⁰⁵⁶) und pKe#83-reverse-17 (¹⁴¹⁸CGGTTCAGCAGCTCATACC¹³⁹⁹) eingesetzt. Es wurden 10 ng cDNA mit je 10 µM Primer zusammen mit einem Gemisch aus hitzestabiler DNA-Polymerase, ATP, TTP, GTP, CTP und Polymerasepuffer (vgl. z.B.: *Current Protocols in Molecular Biology*, Vol. 1, 1997, John Wiley & Sons Inc., Suppl. 37, Chapter 15), hier im Beispiel in Form des im Handel gebrauchsfertig erhältlichen "PCR-Master-Mix" der Firma Clontech, in Ansatz gebracht. Zusätzlich wurden folgende Kontrolluntersuchungen durchgeführt: 1. der vorstehend beschriebene Reaktionsansatz mit dem Plasmid pUEX/pKe#83 anstelle der cDNA ("Positivkontrolle"), 2. der vorstehend beschriebene Reaktionsansatz ohne Zusatz von cDNA ("Negativkontrolle"), 3. der vorstehend beschriebene Reaktionsansatz mit GAPDH-spezifischen Primern (#302047, Stratagene; "GAPDH-Kontrolle").

Die Reaktionsprodukte der PCR-Reaktion wurden im Agarosegel elektrophoretisch aufgetrennt. **Fig. 1.A** zeigt das Ergebnis einer pKe#83-spezifischen PCR Auftrennung. Es gilt:
- Spur 1 =: DNA Molekulargewichtsmarker VII (359-8576 Bb, Boehringer Mannheim)
- Spur 2 =: HaCaT
- Spur 3 =: HMEC (Zellinie, in der pKe#83 nicht nachweisbar ist)
- Spur 4 =: NHEK T0 (direkt nach Ablösung),
- Spur 5 =: NHEK T2 (2 h nach Ablösung)
- Spur 6 =: NHEK T4 (4 h nach Ablösung)
- Spur 7 =: NHEK T8 (8 h nach Ablösung),
- Spur 8 =: pUEX/pKe#83-Plasmid
- Spur 9 =: keine cDNA.

Ein PCR-Produkt der erwarteten Größe von ≈ 390 Bp wurde in den Spuren 2, 5 - 8 nachgewiesen, d.h. pKe#83-spezifische mRNA wurde in den Keratinozytensheets (NHEK) zu den Zeitpunkten 2, 4 und 8 Stunden nach Dispase-induzierter Ablösung und ebenso in HaCaT-Zellen nachgewiesen.

**Fig. 1.B** zeigt das Ergebnis einer GAPDH-spezifischen PCR. Es gilt:
- Spur 1 =: DNA Molekulargewichtsmarker VII (359-8576 Bb, Boehringer Mannheim)
- Spur 2 =: HaCaT
- Spur 3 =: HMEC
- Spur 4 =: NHEK T0
- Spur 5 =: NHEK T2
- Spur 6 =: NHEK T4
- Spur 7 =: NHEK T8

Diese GAPDH-spezifische PCR ("GAPDH-Kontrolle") beweist, daß ein negatives PCR Ergebnis im pKe#83-spezifischen Ansatz nicht auf ein Nichtvorhandensein von cDNA zurückzuführen ist, da in allen Reaktionsansätzen von T0 - T8 ein PCR-Produkt der erwarteten Größe von 600 Bp nachweisbar war.

Die rt-PCR ermöglicht den Nachweis der pKe#83-Expression auch in Fällen, in denen der Nachweis des pKe#83-Proteins aufgrund zu niedrigen Expressionsspiegels mit immunhistologischen Methoden, dem ELISA oder mit Immunoblot-Verfahren nicht gelingt.

### Beispiel 3: Herstellung von Vektormolekülen mit der Fähigkeit zur Expression des Protein pKe#83 in prokaryontischen bzw. eukaryontischen Zellen, sowie Produktion und Reinigung des rekombinanten pKe#83 Proteins

Zur Herstellung bzw. Expression des rekombinanten pKe#83-Proteins wurden zwei Wege beschritten. Zum einen wurde das Vektorkonstrukt pGEX-2T/pKe#83 gemäß Vektorprotokoll in **Fig. 2** für die Expression in Bakterien (E. coli DH5α) hergestellt. Zum anderen wurde das Vektorkonstrukt pcDNA3.1/pKe#83-FLAG gemäß Vektorprotokoll in **Fig. 3**) zum Zweck der Expression in eukaryontischen Zellen (Cos-Zellen) hergestellt.

Das Vektorkonstrukt pGEX-2T/pKe#83 wurde nach Standardprotokollen für die Transformation von E. coli DH5α eingesetzt. Das pKe#83-Glutathion-S-Transferase-(GST)-Fusionsprotein wurde in Bakterien exprimiert, das bakterielle Lysat wurde im Immunoblot mit anti-GST-Antikörpern untersucht, und zwar im Vergleich zu Lysat von Bakterien, die mit einem Kontrollplasmid (kein GST) transformiert wurden.

Das pKe#83/GST-Fusionsprotein wurde durch Affinitätschromatographie mit Hilfe von Gluthathion-Sepharose 4B aus den Bakterienlysaten gereinigt. Die Fraktionen dieser Reinigung wurden dann im Immunoblot mit anti-GST-Antikörpern untersucht.

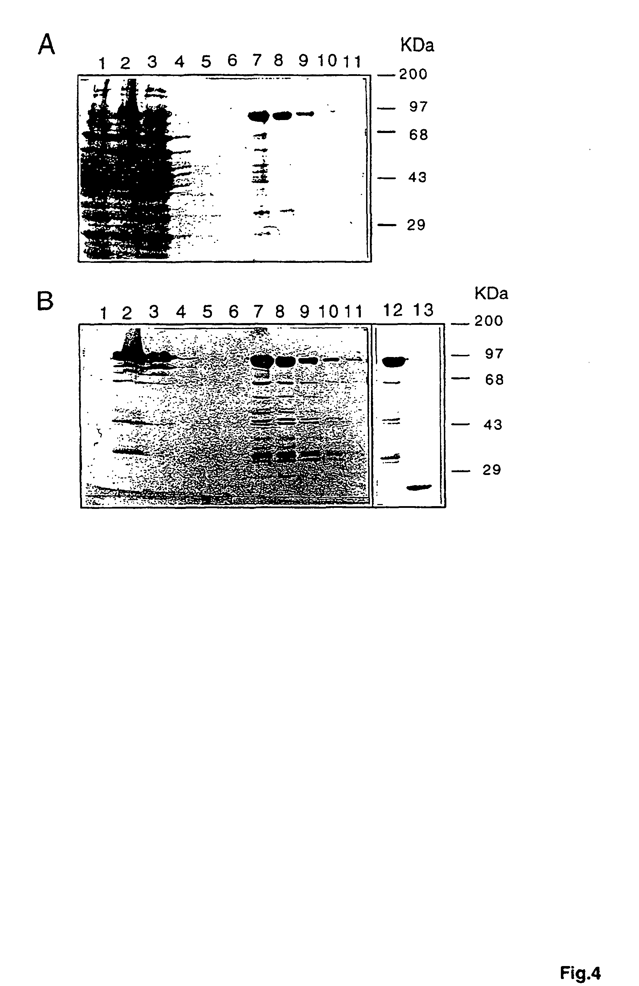

Das Produkt des Immunoblots ist in **Fig. 4.B** abgebildet, **Fig. 4.A** zeigt die entsprechende Proteinfärbung (Ponceau-Rot) des Blots vor Antikörperfärbung. Es gilt:
- Spur 1: = Bakterienlysat der Kontrolltransfektante
- Spur 2 =: Bakterienlysat der pUEX-2T/pKe#83-GST Transfektante
- Spur 3 =: Säulendurchlauf
- Spur 4 - 6 =: Waschfraktion 1-3
- Spur 7-11 =: Elutionsfraktion
- Spur 12 =: pKe#83/GST-Fusionsprotein vor Thrombinverdau
- Spur 13 =: pKe#83/GST-Fusionsprotein nach Thrombinverdau

Das pKe#83/GST-Fusionsproteins hatte ein apparentes Molekulargewicht von ca. 90 KDa. Das erlaubt den Schluß, daß das 90 KDa pKe#83/GST-Fusionsprotein aus dem GST-Protein (ca. 26 kDa) und einem ca. 60 - 65 KDa großen Fragment des Proteins pKe#83 besteht.

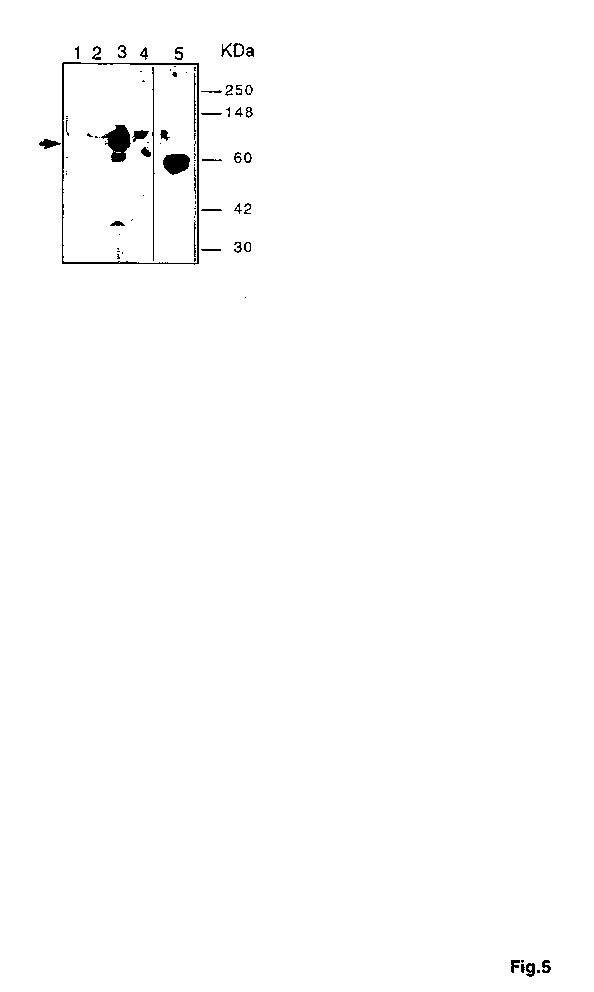

Im eukaryontischen System wurde der pcDNA3.1/pKe#83-FLAG Vektor (**Fig. 3**) in sog. Cos-Zellen, d.h. in Zellen der im Stand der Technik allgemein bekannten Cos-Zellinie, transformiert. Die Zellen wurden nach Standardverfahren durch Behandlung mit DEAE-Dextran/Chloroquin zur Aufnahme der Plasmid-DNA gebracht. Danach wurden die transformierten Zellen drei Tage unter Standardbedingungen (37°C und 7 % CO₂) inkubiert. Die Cos-Zellen wurden lysiert und im Immunoblot unter Verwendung eines Antikörpers gegen das FLAG-Epitop analysiert. **Fig. 5** zeigt das Produkt des Immunoblots:
- Spur 1 =: mit pcDNA3.1/pKe#83-FLAG Vektorkonstrukt transfizierte Cos-Zellen entwickelt mit einem isotyp-identischen Kontroll-Antikörper,
- Spur 2 =: mit dem pcDNA3.1 Vektor (ohne pKe#83) transfizierte Cos-Zellen entwickelt mit einem isotyp-identischen Kontroll-Antikörper,
- Spur 3 =: mit pcDNA3.1/pKe#83-FLAG Vektorkonstrukt transfizierte Cos-Zellen entwickelt mit dem anti-FLAG Antikörper,
- Spur 4 =: mit dem pcDNA3.1 Vektor (ohne pKe#83) transfizierte Cos-Zellen entwickelt mit dem anti-FLAG Antikörper,
- Spur 5 =: FLAG-markiertes Kontrollprotein das die Funktionalität des anti-FLAG-Antikörpers zeigt.

Das Ergebnis dieses Versuch belegt die Expression des pKe#83-FLAG-Fusionsproteins in Cos-Zellen, die mit dem Vektorkonstrukt pcDNA3.1/pKe#83-FLAG transfiziert wurden.

### Beispiel 4: Herstellung und Charakterisierung von Antikörpern gegen das pKe#83 Protein, sowie immunologischer Nachweis des pKe#83 Proteins mittels Immunoblot ("Westernblot"), Immunhistologie und Enzyme-Linked Immunosorbent Assay (ELISA)

Gereinigtes rekombinantes pKe#83-Nichtfusionsprotein wurde für die adjuvanzunterstützte Immunisierung von Kaninchen und Mäusen eingesetzt. Die Details des Immunisierungsverfahrens sind im Stand der Technik allgemein geläufig. Die Immunisierung von Kaninchen wurde im Kundenauftrag von der Fa. *Dr. J. Pineda Antikörper-Service* (Berlin) durchgeführt. Es wurden Seren vor ("Präimmunserum") und nach ("Postimmunserum") Immunisierung gewonnen. Aus den Seren wurde die IgG-Fraktion nach Standardverfahren mittels Ammoniumsulfatfällung isoliert. Die resultierenden IgG-Präparationen werden im folgenden als "anti-pKe#83 IgG" bezeichnet.

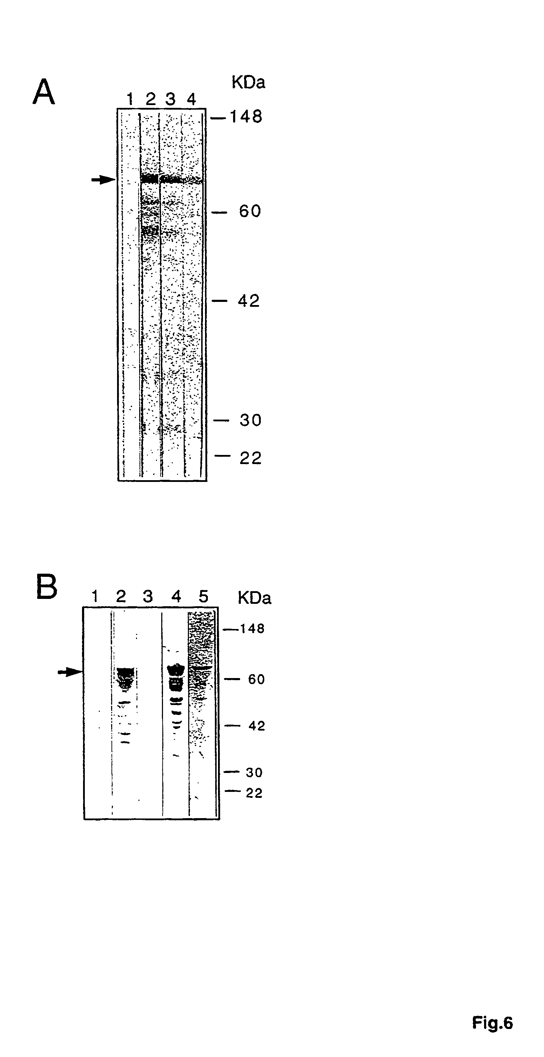

Das Kaninchen "anti-pKe#83 IgG" zeigte eine deutliche Immunreaktion mit dem für die Immunisierung verwendeten rekombinanten pKe#83 Protein. Das Produkt dieses Immunblots ist in **Fig. 6.A** dargestellt. Es gilt:
- Spur 1 =: Präimmun Kaninchen IgG, 1:10 000 verdünnt,
- Spur 2 =: anti-pKe#83 IgG 1:50 000 verdünnt
- Spur 3 =: anti-pKe#83 IgG 1:100 000 verdünnt
- Spur 4 =: anti-pKe#83 IgG 1:200 000 verdünnt

Der Pfeil markiert die Position des pKe#83-Proteins.

Mit dem polyklonalen Kaninchen "anti-pKe#83 IgG" und polyklonalem Maus "anti-pKe#83 IgG" wurden darüberhinaus Zellysate von pKe#83-transfizierten Cos-Zellen im Immunblotverfahren auf die Expression des Proteins pKe#83 getestet. Das Produkt dieses Immunblots ist in **Fig. 6.B** dargestellt. Es gilt:
- Spur 1 =: Präimmun Kaninchen IgG,
- Spur 2 =: Kaninchen "anti-pKe#83 IgG",
- Spur 3 =: normal Maus IgG,
- Spur 4 =: Maus anti-pKe#83 IgG,
- Spur 5 =: anti-FLAG Antikörper.

**Immunhistologie:** Mit Hilfe eines Kryotoms wurden 5 µm-dicke Gefrierschnitte von Geweben aus Hautbiopsien von klinisch unauffälliger Normalhaut und von Dispase-abgelösten NHEK-"Sheets" zum Zeitpunkt T0 und T8 hergestellt. Diese wurden bei Raumtemperatur luftgetrocknet und in 100 % Azeton fixiert (anstelle von Azeton kann ebensogut auch 100 % Methanol, 100 % Ethanol oder 4 %-iges Paraformaldehyd verwendet werden). Danach wurden die Schnitte gemäß im Stand der Technik bekannter sog. "Blockierungsverfahren" behandelt, um unspezifische Bindungsstellen für den Antikörper zu blockieren. Im vorliegenden Beispielfall wurden zwei Blockierungsschritte durchgeführt: (1) eine Blockierung mit Avidin/Biotin und (2) eine Blockierung mit Normalserum. Im ersten Blockierungschritt wurde die Avidin/Biotin-Blockierung unter Verwendung des Avidin/Biotin-Blockierungskits der Firma Vector-Laboratories nach Herstellervorschrift eingesetzt, d.h. es wurde bei Raumtemperatur zunächst 15 Minuten mit der Avidin-Fertiglösung und nachfolgend 15 Minuten mit der mit der Biotin-Fertiglösung inkubiert. Anschließend wurden die Schnitte mit 10 Vol-% Normalserum in PBS für 15 Minuten bei Raumtemperatur inkubiert (Normalserum der Spezies, aus der der Zweitantikörper stammt, hier Ziege-Normalserum, PBS = Phosphate buffered saline = Phosphat-gepufferte Kochsalzlösung, pH 7,2 - 7,4).

Im Anschluß an die Blockierung wurden die Schnitte in PBS mit einem Gehalt an 5µg/ml Kaninchen "anti-pKe#83 IgG" für 1 Stunde bei Raumtemperatur inkubiert. Zur Entfernung des nichtgebundenen Antikörpers wurden die Schnitte anschließend in PBS mit einem Gehalt an 0,2 % (Gewicht/Volumen) bovinem Serumalbumin gewaschen. Es folgt die Inkubation mit einem beispielsweise Biotin-markierten und gegen Kaninchen-IgG-gerichteten Antikörper aus der Ziege (1:500 verdünnt in PBS/ 0,2% BSA; 30 Minuten bei Raumtemperatur), ein weiterer Waschschritt, sowie die Aufbringung eines mit dem Fluoreszenzfarbstoff Cy3-markierten Streptavidins (1 : 1000 in PBS/0,2 % BSA verdünnt). Anstelle von Cy3 kann auch ein anderer Fluoreszenzfarbstoff zur Markierung des Streptavidins verwendet werden, z.B. FITC. Nach einem letzten Waschschritt wurden die Schnitte mit Eindeckmedium, z.B. Elvanol oder Histogel, eingedeckt und im Fluoreszenzmikroskop untersucht und ausgewertet.

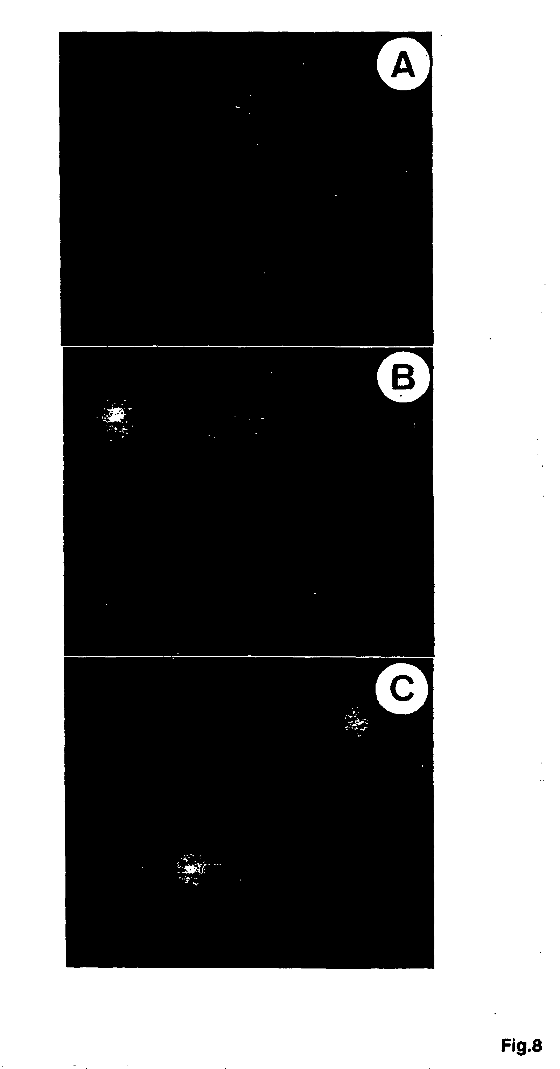

In **Fig. 8** sind die Ergebnisse eines derart durchgeführten Immunfluoreszenztests dargestellt: Das Kaninchen "anti-pKe#83 IgG" zeigt auf Normalhautschnitten eine schwache intrazelluläre und eine starke Zellmembran-assoziierte Immunfärbung (**Fig. 8.C**). Die NHEK "Sheets" T0 (= direkt nach Dispase-induzierter Ablösung vom Substrat) zeigen nur eine leichte Hintergrundfärbung (**Fig. 8.A**) während die NHEK "Sheets" T8 (= 8 Stunden nach der Dispase-induzierten Ablösung vom Substrat) eine deutliche Immunfärbung aufweisen (**Fig. 8.B**). Dieses Ergebnis beinhaltet die Aussage, daß direkt nach der Ablösung wenig Protein pKe#83 vorhanden oder jedenfalls nachweisbar war, aber 8 Stunden später bereits eine vermehrte Expression stattgefunden hatte und infolgedessen deutlich höhere Mengen Protein pKe#83 nachgewiesen werden konnten.

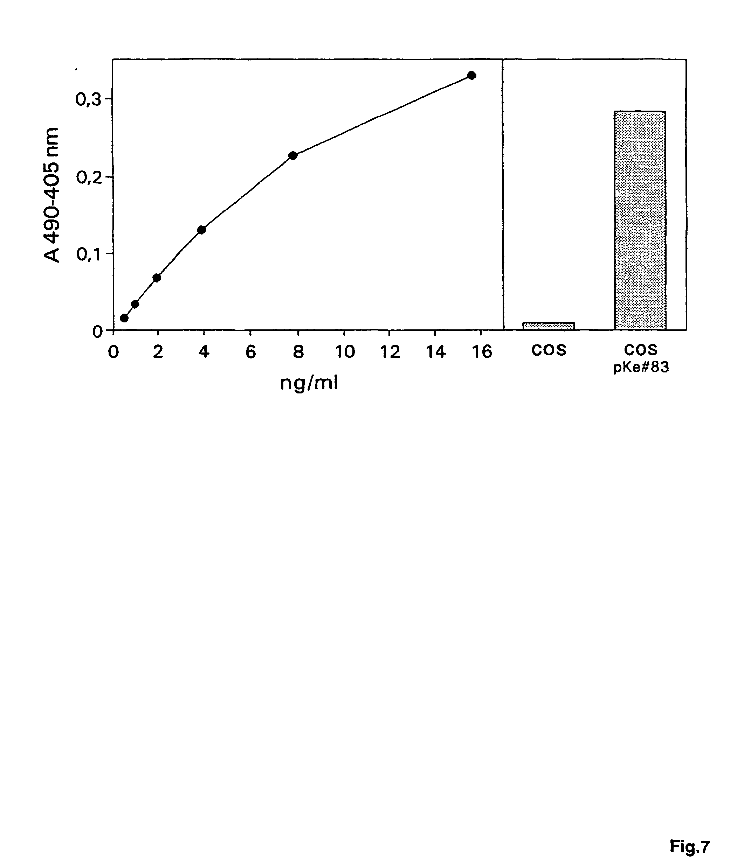

**Enzyme-linked Immunosorbent Assay (ELISA):** Zur Quantifizierung des pKe#83-Proteins in komplexen Lösungen wurde ein sog. "Sandwich"-ELISA **(Fig. 7)** durchgeführt. Hierzu wurde eine Mikrotiterplatte mit einem gegen pKe#83 gerichteten Antikörper (z.B. Kaninchen anti-pKe#83 IgG, 1 µg/Vertiefung) beschichtet. Dann wurden die noch verbliebenen unspezifischen Bindungsstellen der Mikrotiterplatte durch Behandlung mit 0,1 Gewichts-% Gelatine in phospatgepufferter Kochsalzlösung ("PBS/Gelatine") blockiert. Anschließend wurde die Mikrotiterplatte mit unterschiedlichen Konzentration des pKe#83-Proteins als Kalibrator, bzw. mit Verdünnungen unbekannter Proben (in denen die pKe#83-Konzentration festgestellt werden sollte) in Ansatz gebracht. Nach einem Waschschritt mit 0,05 Volumen-% Tween-20 in PBS (PBS/Tween) wurde die Platte mit einer IgG Präparation aus einer zweiten Spezies (z.B. mit Maus anti-pKe#83 IgG) inkubiert (z.B. eine Stunde unter Schütteln bei Raumtemperatur). Nach einem weiteren Waschschritt mit PBS/Tween wurde die Platte mit einer Peroxidase-markierten kommerziellen Kaninchen anti Maus-IgG Antikörper-Präparation inkubiert (z.B. Fc-spez. Fab₂-POX von Dianova GmbH, Hamburg). "Peroxidase" steht hier stellvertretend für praktisch jede beliebige Markierung des Antikörpers, z.B. mit Enzymen, Fluoreszenzmolekülen oder Lumineszenzmolekülen. Nach einem weiteren Waschschritt zur Entfernung ungebundener enzymmarkierter Antikörper wurde das farblose Peroxidase-Substrat Ortho-Phenylendiamin zugesetzt, welches durch die Peroxidase-Aktivität in ein farbiges Produkt umgewandelt wird. Die Quantifizierung der Farbbildung erfolgt in einem Mikrotiterplattenphotometer bei 490 nm gegen 405 nm (Ordinate).

Das Ergebnis des Versuchs ist in **Fig. 7** dargestellt. Es zeigt, daß die Farbkonzentration (angegeben als Absorption in der Ordinate) der Menge des eingesetzten pKe#83-Proteins (= des "Kalibrators", in der Abszisse dargestellt) proportional ist. Um die Funktionalität des Testsystems zu demonstrieren, wurden gleichzeitig Lysate von zwei verschiedenen Cos-Transfektanten-Ansätzen getestet, die sich in der Expression von pKe#83 unterscheiden. Die Cos-Zellen des einen Ansatzes wurden mit dem Vektorkonstrukt pcDNA3.1/pKe#83 (Ansatz "Cos pKe#83") und die des anderen Ansatzes mit dem pcDNA3.1 Vektor ohne Insert (Ansatz "Cos") transfiziert.

Zellen dieser Transfektanten-Ansätze wurden nach Standardverfahren unter Verwendung des Detergenzes Triton X-100 lysiert. Diese Lysate wurden in einer 1:10-Verdünnung in PBS/Tween 20 im ELISA getestet. Lysate des "Cos pKe#83"-Transfektanten-Ansatzes zeigten eine positive Reaktion zeigen. Unter Berücksichtigung der Kalibratordaten wurde eine Konzentration von ca. 120 ng pKe#83/ 10⁶ Cos pKe#83-Zellen festgestellt. Bei den Lysaten der Kontroll-Transfektanten-Ansätze "Cos" konnte kein Protein pKe#83 nachgewiesen werden. Durch den Einsatz dieses Testverfahrens ist folglich eine Quantifizierung einer unbekannten Menge des Proteins pKe#83 in einer Probe möglich.

Die Substanz Ortho-Phenylendiamin steht hier stellvertretend für jedes beliebige Peroxidase-Substrat, das infolge der Peroxidase-Aktivität seine Farbe nachweisbar verändert. Anstelle der hier beispielhaft verwendeten polyklonalen Antikörper können ebensogut monoklonale Antikörper, die gegen das Protein pKe#83 gerichtet sind, eingesetzt werden. Anstatt des indirekten Ansatzes über einen markierten speziesspezifischen anti-IgG Antikörper kann auch die Durchführung mit einem direkt markierten antipKe#83-Antikörper erfolgen.

### Beispiel 5: Beeinflussung von Keratinozyten durch pKe#83-spezifische Oligonukleotide

Antisense-Oligonukleotide werden von Zellen, auch Keratinozyten, aufgenommen (vgl.: *G. Hartmann et al*. *1998: Antisense-Oligonukleotide, Deutsches Ärzteblatt 95, Heft 24, C1115 - C1119*). Sie binden in spezifischer Weise an die in der Zelle vorliegende mRNA und hemmen deren Translation und damit die Expression des entsprechenden Proteins (vgl.: *Y.-S. Lee et al. 1997*, *Definition by specific antisense oligonucleotides of a role for protein kinase Cα in expression of differentiation markers in normal and neoplastic mouse epidermal keratinocytes, Molecular Carcinogenesis 18: 44-53*). Geeignete Antisense-Oligonukleotide wurden anhand der pKe#83-spezifischen Nukleotidsequenz (**SEQ ID NO: 1 bzw. SEQ ID NO: 7**) hergestellt. Sie wurden mit geeignetem Puffermedium (sog. "Oligopuffer") auf eine Konzentration von 100 µM eingestellt. HaCaT-Zellen wurden bei 37°C und 7 % CO₂ bis zu einer Konfluenz von 70 - 80 % kultiviert. Die Zellen wurden abtrypsiniert (10 Minuten 0,2 Gewichts-% EDTA, 0,1 Gewichts-% Trypsin, 5 - 10 Minuten) und auf eine Konzentration von 25 000 Zellen/ml eingestellt. Pro Vertiefung einer Mikrotiter-Kulturplatte (96 Vertiefungen) wurden 100 µl Zellsuspension (entspricht 2500 Zellen) einpipepttiert. Die Zellen wurden 1 Stunde unter den vorgenannten Kultivierungsbedingungen inkubiert, danach erfolgte die Zugabe des Antisense-Oligonukleotids (2 µl einer 100 µM-Lösung) und eine weitere Inkubation von 24 - 48 Stunden. Als Negativkontrolle dienten Zellansätze, denen Oligonukleotide mit der gleichen Basenverteilung aber zufällig ausgewählter Sequenz zugegeben wurden.

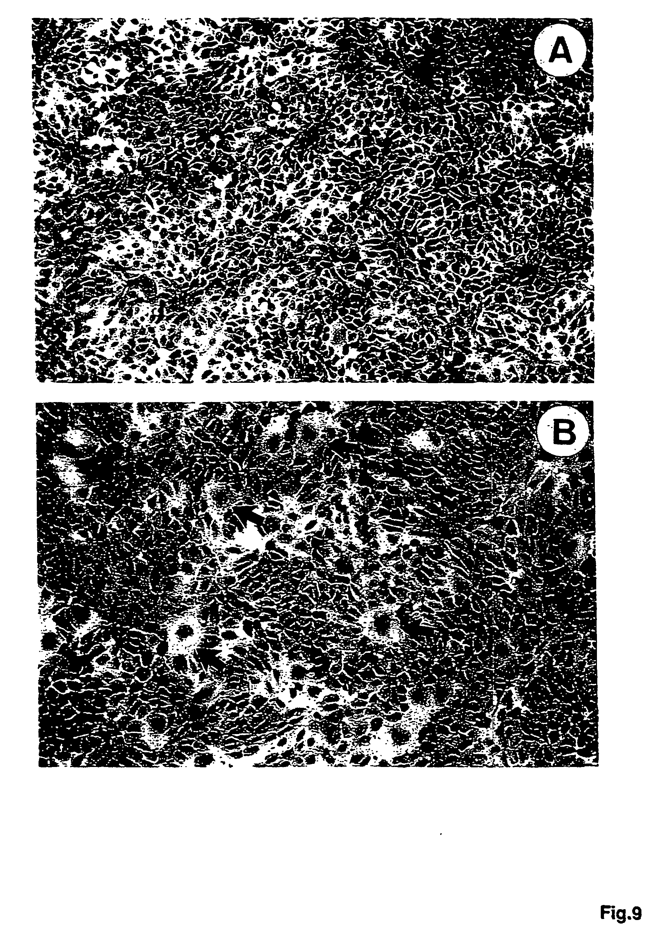

Die solcherart behandelten Zellen wurden mit Hilfe eines Mikroskops hinsichtlich phänotypischer Veränderungen untersucht. Das Ergebnis der mikroskopischen Analyse ist in **Fig. 9** dargestellt: **Fig. 9.A** zeigt HaCaT-Zellen, die mit Kontroll-Oligonukleotiden behandelt, worden sind, **Fig. 9.B** zeigt HaCaT-Zellen, die mit pKe#83-spezifischen Antisense-Oligonukleotiden behandelt worden sind.

Die mikroskopischen Untersuchungen zeigten, daß in den mit Antisense-Oligonukleotiden behandelten HaCaT-Kulturen stark vergrößerte Zellen auftraten (**Fig. 9.B,** Pfeile), die in den mit Kontroll-Oligonukleotiden behandelten Kulturen nicht zu finden waren. Diese großen Zellen entsprechen in ihrer Morphologie differenzierten Keratinozyten. Der Befund weist darauf hin, daß mit pKe#83-spezifischen Antisense-Oligonukleotiden behandelte Zellen eine vermehrte Tendenz zur Differenzierung aufweisen.

### SEQUENZPROTOKOLLE

### ANZAHL DER SEQUENZEN: 8

COMPUTERLESBARE FASSUNG:
   - DATENTRÄGER:: Diskette
   - COMPUTER:: IBM-kompatibler PC
   - BETRIEBSSYSTEM:: MS-DOS
   - SOFTWARE:: Microsoft WORD für Windows 97

**ANGABEN ZU SEQ ID NO:1:**
   SEQUENZCHARAKTERISTIKA:
      - LÄNGE:: 2667 Basenpaare
      - ART:: Desoxyribonukleinsäure
      - TOPOLOGIE:: linear
   ART DES MOLEKÜLS: cDNA
   HYPOTHETISCH: nein
   ANTI-SENSE: nein
   URSPRÜNGLICHE HERKUNFT:
      - ORGANISMUS:: Homo sapiens
      - STAMM:: kaukasisch
      - ENTWICKLUNGSSTADIUM:: adult
      - ZELLTYP:: epidermaler Keratinozyt
   UNMITTELBARE HERKUNFT: cDNA aus Keratinozyten
   MERKMAL:
      - NAME/SCHLÜSSEL:: kodierende Sequenz für regulatorisches Protein pKe#83 aus humanen Keratinozyten
      - LAGE:: von 1 bis 2667
      - ERMITTLUNGSMETHODE:: cDNA-Sequenzierung 
SEQ ID NO: 1

1 GTTTTGTTAG GCAAAAAGAG ACTATTGAAA GCTGAGACTT TAGAATTGAG
51 TGACTTATAT GTTAGTGATA AGAAGAAGGA TATGTCTCCA CCCTTTATTT
101 GTGAGGAGAC AGATGAACAA AAGCTTCAAA CTCTAGACAT CGGTAGTAAC
151 TTGGAGAAAG AAAAATTAGA GAATTCCAGA TCCTTAGAAT GCAGATCAGA
201 TCCAGAATCT CCTATCAAAA AAACAAGTTT ATCTCCTACT TCTAAACTTG
251 GATACTCATA TAGTAGAGAT CTAGACCTTG CTAAGAAAAA ACATGCTTCC
301 CTGAGGCAGA CGGAGTCTGA TCCAGATGCT GATAGAACCA CTTTAAATCA
351 TGCAGATCAT TCATCAAAAA TAGTCCAGCA TCGATTGTTA TCTAGACAAG
401 AAGAACTTAA GGAAAGAGCA AGAGTTCTGC TTGAGCAAGC AAGAAGAGAT
451 GCAGCCTTAA AGGCGGGGAA TAAGCACAAT ACCAACACAG CCACCCCATT
501 CTGCAACAGG CAGCTAAGTG ATCAGCAAGA TGAAGAGCGA CGTCGGCAGC
551 TGAGAGAGAG AGCTCGTCAG CTAATAGCAG AAGCTCGATC TGGAGTGAAG
601 ATGTCAGAAC TTCCCAGCTA TGGTGAAATG GCTGCAGAAA AGTTGAAAGA
651 AAGGTCAAAG GCATCTGGAG ATGAAAATGA TAATATTGAG ATAGATACTA
 
701 ACGAGGAGAT CCCTGAAGGC TTTGTTGTAG GAGGTGGAGA TGAACTTACT
751 AACTTAGAAA ATGACCTTGA TACTCCCGAA CAAAACAGTA AGTTGGTGGA
801 CTTGAAGCTG AAGAAGCTCC TAGAAGTTCA GCCACAGGTG GCAAATTCAC
851 CCTCCAGTGC TGCCCAGAAA GCTGTAACTG AGAGCTCAGA GCAGGACATG
901 AAAAGTGGCA CAGAAGATCT CCGGACTGAA CGATTACAAA AAACAACAGA
951 ACGTTTTAGA AATCCTGTTG TGTTCAGCAA AGATTCTACA GTCAGAAAAA
1001 CTCAACTTCA GTCTTTCAGC CAATATATTG AGAATAGACC AGAGATGAAA
1051 AGGCAGAGAT CAATACAGGA AGATACAAAG AAAGGAAATG AGGAGAAGGC
1101 AGCGATAACT GAAACTCAGA GGAAGCCATC AGAAGATGAA GTGCTTAATA
1151 AAGGGTTCAA AGACACCAGT CAGTATGTAG TAGGAGAATT GGCAGCACTA
1201 GAGAATGAGC AAAAGCAAAT TGACACCCGT GCCGCGCTGG TGGAGAAGCG
1251 CCTTCGCTAT CTCATGGACA CAGGAAGGAA CACAGAAGAA GAAGAAGCTA
1301 TGATGCAGGA ATGGTTTATG TTAGTTAATA AGAAAAATGC CTTAATAAGG
1351 AGAATGAATC AGCTCTCTCT TCTGGAAAAA GAACATGATT TAGAACGACG
1401 GTATGAGCTG CTGAACCGGG AATTGAGGGC AATGCTAGCC ATTGAAGACT
1451 GGCAGAAGAC CGAGGCCCAG AAGCGACGCG AACAGCTTCT GCTAGATGAG
1501 CTGGTGGCCC TGGTGAACAA GCGCGATGCG CTCGTCAGGG ACCTGGACGC
1551 GCAGGAGAAG CAGGCCGAAG AAGAAGATGA GCATTTGGAG CGAACTCTGG
1601 AGCAAAACAA AGGCAAGATG GCCAAGAAAG AGGAGAAATG TGTTCTTCAG
1651 TAGCCATCAG ATCAGAAAGA ATCTCTCCCA ACATTTTAGA GTCTTGCTTC
1701 CCAAACCAGA AAAAGTCAGA CTCATTGTTG ATTTAAAACT TTTAACATTT
1751 TGTTTGGCTG GATTGTACTA CTTTACCTCT ACTTTACCAC CACCACCCTT
1801 TTCCTCCCTC CTTTCCAAAT AATATACAGA ACTCCAAAAT AGCTTCATTT
1851 AAGGATTTTT TTGTGAGTTA ACAATTTCCT TGAAATCCTG TGAAATAGAT
1901 TTGCACAGAC ACCTTGTGAG TGATTGGTAT TGGAGGTGTT CAAGAAACTG
1951 TTCGAAAAAG AACAAAAACA CTTCCCTCGT TATTTTCTCT CATTTTTTGA
2001 TGAGAGGAAA ATTTGAAACA TTATTCTTGT TGTTGTTGGT AATAGCATAA
2051 TGACAGTGGG AGGGGTACAA GGGGATAAGA AAAATGTCAT GATTTTTTTC
2101 CGGTCCTGCC ACATGTAACA CTTACTCTGT TACCTAAATT TTATAGTTAG
2151 ATCATATCCA ATCTACTTAT TAAACTGTGT TCTATTTACC AGTGGAGTTT
2201 TTCTGCAGTG GTTGCGTTTC ACTGTAAGGA TAATGGAGTT CCTCTCCTCT
2251 GCTTTCCTCA GAGGATGGTC CTTTAACATA GCCAGAAACA AGCCCTGTGG
2301 TTTGAAGGTG AGCTGTGAGG ATGGGACTAA TTGATATGCA CCAGTTTACA
2351 AAGACAGTCT TATCATCCGA GAATACACCA TCTTTTTCTC TGGATAATTA
2401 TTTCTTACAT CATGCTTGAT TCCTACATTT TGTTGGGTTT CAACATTGGC
2451 TCACGAATGC TGTTAATATT TATTCTGTAT TGATAAAAAG TCTGTCTTGC
2501 CACTACAAGT AAATCCCCCA TTTAATATTT TCTTCTTTAG CATAGCACTG
2551 TCATTTTTTG TGAAAATGGT TATGTTTATT TATTACAATA CTGAGTCATA
2601 TATAAATTTT CAATAAAAGC AGAAACTTTC TTACCTTAAA AAAAAAAAAA
2651 AAAAAAAAAA AAAAAAA
**ANGABEN ZU SEQ ID-NO:2**
   SEQUENZCHARAKTERISTIKA:
      - LÄNGE:: 2547 Basenpaare
      - ART:: Desoxyribonukleinsäure
      - TOPOLOGIE:: linear
   ART DES MOLEKÜLS: cDNA
   HYPOTHETISCH: nein
   ANTI-SENSE: nein
   URSPRÜNGLICHE HERKUNFT:
      - ORGANISMUS:: Homo sapiens
      - STAMM:: kaukasisch
      - ENTWICKLUNGSSTADIUM:: adult
      - ZELLTYP:: epidermaler Keratinozyt
   UNMITTELBARE HERKUNFT: cDNA aus Keratinozyten
   MERKMAL:
      - NAME/SCHLÜSSEL:: Splice-Variante einer kodierende Sequenz für das regulatorische Protein pKe#83 aus humanen Keratinozyten
      - LAGE:: von 1 bis 2547
      - ERMITTLUNGSMETHODE:: cDNA-Sequenzierung 
SEQ ID NO: 2

1 GTTTTGTTAG GCAAAAAGAG ACTATTGAAA GCTGAGACTT TAGAATTGAG
51 TGACTTATAT GTTAGTGATA AGAAGAAGGA TATGTCTCCA CCCTTTATTT
101 GTGAGGAGAC AGATGAACAA AAGCTTCAAA CTCTAGACAT CGGTAGTAAC
151 TTGGAGAAAG AAAAATTAGA GAATTCCAGA TCCTTAGAAT GCAGATCAGA
201 TCCAGAATCT CCTATCAAAA AAACAAGTTT ATCTCCTACT TCTAAACTTG
251 GATACTCATA TAGTAGAGAT CTAGACCTTG CTAAGAAAAA ACATGCTTCC
301 CTGAGGCAGA CGGAGTCTGA TCCAGATGCT GATAGAACCA CTTTAAATCA
351 TGCAGATCAT TCATCAAAAA TAGTCCAGCA TCGATTGTTA TCTAGACAAG
401 AAGAACTTAA GGAAAGAGCA AGAGTTCTGC TTGAGCAAGC AAGAAGAGAT
451 GCAGCCTTAA AGGCGGGGAA TAAGCACAAT ACCAACACAG CCACCCCATT
501 CTGCAACAGG CAGCTAAGTG ATCAGCAAGA TGAAGAGCGA CGTCGGCAGC
551 TGAGAGAGAG AGCTCGTCAG CTAATAGCAG AAGCTCGATC TGGAGTGAAG
601 ATGTCAGAAC TTCCCAGCTA TGGTGAAATG GCTGCAGAAA AGTTGAAAGA
651 AAGGTCAAAG CAAAACAGTA AGTTGGTGGA CTTGAAGCTG AAGAAGCTCC
701 TAGAAgTTCA gCCACAGGTG GCAAATTCaC CCTCCAGTGC TGCCCAGAAA
751 GCTGTAACTG AgAgCTCaGA gCaGGACATG AAAAGTGGCa CAGAAGATCT
801 CCGGACTGAA CGATTACAAA AAACAACAGA ACGTTTTAGA AATCCTGTTG
 
851 TGTTCAGCAA AGATTCTACA GTCAGAAAAA CTCAACTTCA GTCTTTCAGC
901 CAATATATTG AGAATAGACC AGAGATGAAA AGGCAGAGAT CAATACAGGA
951 AGATACAAAG AAAGGAAATG AGGAGAAGGC AGCGATAACT GAAACTCAGA
1001 GGAAGCCATC AGAAGATGAA GTGCTTAATA AAGGGTTCAA AGACACCAGT
1051 CAGTATGTAG TAGGAGAATT GGCAGCACTA GAGAATGAGC AAAAGCAAAT
1101 TGACACCCGT GCCGCGCTGG TGGAGAAGCG CCTTCGCTAT CTCATGGACA
1151 CAGGAAGGAA CACAGAAGAA GAAGAAGCTA TGATGCAGGA ATGGTTTATG
1201 TTAGTTAATA AGAAAAATGC CTTAATAAGG AGAATGAATC AGCTCTCTCT
1251 TCTGGAAAAA GAACATGATT TAGAACGACG GTATGAGCTG CTGAACCGGG
1301 AATTGAGGGC AATGCTAGCC ATTGAAGACT GGCAGAAGAC CGAGGCCCAG
1351 AAGCGACGCG AACAGCTTCT GCTAGATGAG CTGGTGGCCC TGGTGAACAA
1401 GCGCGATGCG CTCGTCAGGG ACCTGGACGC GCAGGAGAAG CAGGCCGAAG
1451 AAGAAGATGA GCATTTGGAG CGAACTCTGG AGCAAAACAA AGGCAAGATG
1501 GCCAAGAAAG AGGAGAAATG TGTTCTTCAG TAGCCATCAG ATCAGAAAGA
1551 ATCTCTCCCA ACATTTTAGA GTCTTGCTTC CCAAACCAGA AAAGTCAGA
1601 CTCATTGTTG ATTTAAAACT TTTAACATTT TGTTTGGCTG GATTGTACTA
1651 CTTTACCTCT ACTTTACCAC CACCACCCTT TTCCTCCCTC CTTTCCAAAT
1701 AATATACAGA ACTCCAAAAT AGCTTCATTT AAGGATTTTT TTGTGAGTTA
1751 ACAATTTCCT TGAAATCCTG TGAAATAGAT TTGCACAGAC ACCTTGTGAG
1801 TGATTGGTAT TGGAGGTGTT CAAGAAACTG TTCGAAAAAG AACAAAAACA
1851 CTTCCCTCGT TATTTTCTCT CATTTTTTGA TGAGAGGAAA ATTTGAAACA
1901 TTATTCTTGT TGTTGTTGGT AATAGCATAA TGACAGTGGG AGGGGTACAA
1951 GGGGATAAGA AAAATGTCAT GATTTTTTTC CGGTCCTGCC ACATGTAACA
2001 cTTAcTcTGT TACCTAAATT TTATAGTTAG ATCATATCCa ATctACTTAT
2051 TAAACTGTGT TCTATTTACC AGTGGAGTTT TTcTGCAGTG GtTGCGTTTC
2101 ACTGTAAGGA TAATGGAGTT CcTcTCcTCT GCTTTCCTCA GAGGATGGTC
2151 CTTtAAcATA GCCAGAAACA AGCCCTGTGG TTTGAAGGTG AGCTGTGAGG
2201 ATGGGACTAA TTGATATGCA CCAGTTtACA AAGACAGTcT TaTCATCCGA
2251 GAAtACACCA TcTTTTTcTc TGGATAATTA TTTCTtACAT CATGCTTGAT
2301 TCCtACATTT TGTTGGGTTT CAACATTGGC TCACGAATGC TGTTAAtATT
2351 TATTCTGTAT tGATAAAAAG TcTGTcTTGC CACtACAAGT AAATCCCCCA
2401 TTTAATATTT TcTTcTTTAG CATAGCACTG TCATTTTTTG TGAAAATGGT
2451 TATGTTTATT TATTACAATA CTGAGTCATA TATAAATTTT CAATAAAAGC
2501 AGAAACTTTC TTACCTTAAA AAAAAAAAAA AAAAAAAAAA AAAAAAA
**ANGABEN ZU SEQ ID NO: 3**
   SEQUENZCHARAKTERISTIKA:
      - LÄNGE:: 523 Aminosäuren
      - ART:: Aminosäuresequenz
   ART DES MOLEKÜLS: Protein
   URSPRÜNGLICHE HERKUNFT:
      - ORGANISMUS:: Homo sapiens
      - STAMM:: kaukasisch
      - ENTWICKLUNGSSTADIUM:: adult
      - ZELLTYP:: epidermaler Keratinozyt
   UNMITTELBARE HERKUNFT: abgeleitet aus cDNA-Sequenz
   MERKMAL:
      - NAME/SCHLÜSSEL:: kodierende Sequenz für das regulatorische Protein pKe#83 aus humanen Keratinozyten
      - LAGE:: von 1 bis 523
      - ERMITTLUNGSMETHODE:: Ableitung aus cDNA-Sequenz
      - SONSTIGE ANGABEN:: umfaßt eine Prenyl-Gruppen-Bindungsstelle ("CAAX-Box"), neun Proteinkinasephosphorylierungsmotive, 15 Caseinkinasephosphory lierungsmotive und zwei Tyrosinkinasephosphorylierungsmotive 
SEQ ID NO: 3

1 MSPPFICEET DEQKLQTLDI GSNLEKEKLE NSRSLECRSD PESPIKKTSL
51 SPTSKLGYSY SRDLDLAKKK HASLRQTESD PDADRTTLNH ADHSSKIVQH
101 RLLSRQEELK ERARVLLEQA RRDAALKAGN KHNTNTATPF CNRQLSDQQD
151 EERRRQLRER ARQLIAEARS GVKMSELPSY GEMAAEKLKE RSKASGDEND
201 NIEIDTNEEI PEGFVVGGGD ELTNLENDLD TPEQNSKLVD LKLKKLLEVQ
251 PQVANSPSSA AQKAVTESSE QDMKSGTEDL RTERLQKTTE RFRNPVVFSK
301 DSTVRKTQLQ SFSQYIENRP EMKRQRSIQE DTKKGNEEKA AITETQRKPS
351 EDEVLNKGFK DTSQYVVGEL AALENEQKQI DTRAALVEKR LRYLMDTGRN
401 TEEEEAMMQE WFMLVNKKNA LIRRMNQLSL LEKEHDLERR YELLNRELRA
451 MLAIEDWQKT EAQKRREQLL LDELVALVNK RDALVRDLDA QEKQAEEEDE
501 HLERTLEQNK GKMAKKEEKC VLQ*
**ANGABEN ZU SEQ ID-NO:4:**
   SEQUENZCHARAKTERISTIKA:
      - LÄNGE:: 482 Aminosäuren
      - ART:: Aminosäuresequenz
   ART DES MOLEKÜLS: Protein
   URSPRÜNGLICHE HERKUNFT:
      - ORGANISMUS:: Homo sapiens
      - STAMM:: kaukasisch
      - ENTWICKLUNGSSTADIUM:: adult
      - ZELLTYP:: epidermaler Keratinozyt
   UNMITTELBARE HERKUNFT: abgeleitet aus cDNA-Sequenz
   MERKMAL:
      - NAME/SCHLÜSSEL:: kodierende Sequenz für das regulatorische Protein pKe#83 aus humanen Keratinozyten
      - LAGE:: von 1 bis 482
      - ERMITTLUNGSMETHODE:: Ableitung aus cDNA-Sequenz
      - SONSTIGE ANGABEN:: umfaßt eine Prenyl-Gruppen-Bindungsstelle ("CAAX-Box"), neun Proteinkinasephosphorylierungsmotive, 15 Caseinkinasephosphory lierungsmotive und zwei Tyrosinkinasephosphorylierungsmotive 
SEQ ID NO: 4

1 MSPPFICEET DEQKLQTLDI GSNLEKEKLE NSRSLECRSD PESPIKKTSL
51 SPTSKLGYSY SRDLDLAKKK HASLRQTESD PDADRTTLNH ADHSSKIVQH
101 RLLSRQEELK ERARVLLEQA RRDAALKAGN KHNTNTATPF CNRQLSDQQD
151 EERRRQLRER ARQLIAEARS GVKMSELPSY GEMAAEKLKE EQNSKLVDLK
201 LKKLLEVQPQ VANSPSSAAQ KAVTESSEQD MKSGTEDLRT ERLQKTTERF
251 RNPVVFSKDS TVRKTQLQSF SQYIENRPEM KRQRSIQEDT KKGNEEKAAI
301 TETQRKPSED EVLNKGFKDT SQYVVGELAA LENEQKQIDT RAALVEKRLR
351 YLMDTGRNTE EEEAMMQEWF MLVNKKNALI RRMNQLSLLE KEHDLERRYE
401 LLNRELRAML AIEDWQKTEA QKRREQLLLD ELVALVNKRD ALVRDLDAQE
451 KQAEEEDEHL ERTLEQNKGK MAKKEEKCVL Q*
**ANGABEN ZU SEQ ID NO: 5**
   SEQUENZCHARAKTERISTIKA:
      - LÄNGE:: 2559 Basenpaare
      - ART:: Desoxyribonukleinsäure
      - TOPOLOGIE:: linear
   ART DES MOLEKÜLS: cDNA
   HYPOTETISCH: nein
   ANTI-SENSE: nein
   URSPRÜNGLICHE HERKUNFT:
      - ORGANISMUS:: Homo Sapiens
      - STAMM:: kaukasisch
      - ENTWICKLUNGSSTADIUM:: adult
      - ZELLTYP:: epidermaler Keratinozyt
   UNMITTELBARE HERKUNFT: cDNA aus Keratinozyten
   MERKMAL:
      - NAME/SCHLÜSSEL:: Splice Variante einer kodierenden Sequenz für das regulatorische Protein pKe#83 aus humanen Keratinozyten
      - LAGE:: von 1 bis 2559
      - ERMITTLUNGSMETHODE:: cDNA-Sequenzierung 
SEQ ID NO: 5

1 GTTTTGTTAG GCAAAAAGAG ACTATTGAAA GCTGAGACTT TAGAATTGAG
51 TGACTTATAT GTTAGTGATA AGAAGAAGGA TATGTCTCCA CCCTTTATTT
101 GTGAGGAGAC AGATGAACAA AAGCTTCAAA CTCTAGACAT CGGTAGTAAC
151 TTGGAGAAAG AAAAATTAGA GAATTCCAGA TCCTTAGAAT GCAGATCAGA
201 TCCAGAATCT CCTATCAAAA AAACAAGTTT ATCTCCTACT TCTAAACTTG
251 GATACTCATA TAGTAGAGAT CTAGACCTTG CTAAGAAAAA ACATGCTTCC
301 CTGAGGCAGA CGGAGTCTGA TCCAGATGCT GATAGAACCA CTTTAAATCA
351 TGCAGATCAT TCATCAAAAA TAGTCCAGCA TCGATTGTTA TCTAGACAAG
401 AAGAACTTAA GGAAAGAGCA AGAGTTCTGC TTGAGCAAGC AAGAAGAGAT
451 GCAGCCTTAA AGGCGGGGAA TAAGCACAAT ACCAACACAG CCACCCCATT
501 CTGCAACAGG CAGCTAAGTG ATCAGCAAGA TGAAGAGCGA CGTCGGCAGC
551 TGAGAGAGAG AGCTCGTCAG CTAATAGCAG AAGCTCGATC TGGAGTGAAG
601 ATGTCAGAAC TTCCCAGCTA TGGTGAAATG GCTGCAGAAA AGTTGAAAGA
651 AAGGTCAAAG GCATCTGGAG AACAAAACAG TAAGTTGGTG GACTTGAAGC
701 TGAAGAAGCT CCTAGAAGTT CAGCCACAGG TGGCAAATTC ACCCTCCAGT
751 GCTGCCCAGA AAGCTGTAAC TGAGAGCTCA GAGCAGGACA TGAAAAGTGG
801 CACAGAAGAT CTCCGGACTG AACGATTACA AAAAACAACA GAACGTTTTA
851 GAAATCCTGT TGTGTTCAGC AAAGATTCTA CAGTCAGAAA AACTCAACTT
 
901 CAGTCTTTCA GCCAATATAT TGAGAATAGA CCAGAGATGA AAAGGCAGAG
951 ATCAATACAG GAAGATACAA AGAAAGGAAA TGAGGAGAAG GCAGCGATAA
1001 CTGAAACTCA GAGGAAGCCA TCAGAAGATG AAGTGCTTAA TAAAGGGTTC
1051 AAAGACACCA GTCAGTATGT AGTAGGAGAA TTGGCAGCAC TAGAGAATGA
1101 GCAAAAGCAA ATTGACACCC GTGCCGCGCT GGTGGAGAAG CGCCTTCGCT
1151 ATCTCATGGA CACAGGAAGG AACACAGAAG AAGAAGAAGC TATGATGCAG
1201 GAATGGTTTA TGTTAGTTAA TAAGAAAAAT GCCTTAATAA GGAGAATGAA
1251 TCAGCTCTCT CTTCTGGAAA AAGAACATGA TTTAGAACGA CGGTATGAGC
1301 TGCTGAACCG GGAATTGAGG GCAATGCTAG CCATTGAAGA CTGGCAGAAG
1351 ACCGAGGCCC AGAAGCGACG CGAACAGCTT CTGCTAGATG AGCTGGTGGC
1401 CCTGGTGAAC AAGCGCGATG CGCTCGTCAG GGACCTGGAC GCGCAGGAGA
1451 AGCAGGCCGA AGAAGAAGAT GAGCATTTGG AGCGAACTCT GGAGCAAAAC
1501 AAAGGCAAGA TGGCCAAGAA AGAGGAGAAA TGTGTTCTTC AGTAGCCATC
1551 AGATCAGAAA GAATCTCTCC CAACATTTTA GAGTCTTGCT TCCCAAACCA
1601 GAAAAAGTCA GACTCATTGT TGATTTAAAA CTTTTAACAT TTTGTTTGGC
1651 TGGATTGTAC TACTTTACCT CTACTTTACC ACCACCACCC TTTTCCTCCC
1701 TCCTTTCCAA ATAATATACA GAACTCCAAA ATAGCTTCAT TTAAGGATTT
1751 TTTTGTGAGT TAACAATTTC CTTGAAATCC TGTGAAATAG ATTTGCACAG
1801 ACACCTTGTG AGTGATTGGT ATTGGAGGTG TTCAAGAAAC TGTTCGAAAA
1851 AGAACAAAAA CACTTCCCTC GTTATTTTCT CTCATTTTTT GATGAGAGGA
1901 AAATTTGAAA CATTATTCTT GTTGTTGTTG GTAATAGCAT AATGACAGTG
1951 GGAGGGGTAC AAGGGGATAA GAAAAATGTC ATGATTTTTT TCCGGTCCTG
2001 CCACATGTAA CACTTACTCT GTTACCTAAA TTTTATAGTT AGATCATATC
2051 CAATCTACTT ATTAAACTGT GTTCTATTTA CCAGTGGAGT TTTTCTGCAG
2101 TGGTTGCGTT TCACTGTAAG GATAATGGAG TTCCTCTCCT CTGCTTTCCT
2151 CAGAGGATGG TCCTTTAACA TAGCCAGAAA CAAGCCCTGT GGTTTGAAGG
2201 TGAGCTGTGA GGATGGGACT AATTGATATG CACCAGTTTA CAAAGACAGT
2251 CTTATCATCC GAGAATACAC CATCTTTTTC TCTGGATAAT TATTTCTTAC
2301 ATCATGCTTG ATTCCTACAT TTTGTTGGGT TTCAACATTG GCTCACGAAT
2351 GCTGTTAATA TTTATTCTGT ATTGATAAAA AGTCTGTCTT GCCACTACAA
2401 GTAAATCCCC CATTTAATAT TTTCTTCTTT AGCATAGCAC TGTCATTTTT
2451 TGTGAAAATG GTTATGTTTA TTTATTACAA TACTGAGTCA TATATAAATT
2501 TTCAATAAAA GCAGAAACTT TCTTACCTTA AAAAAAAAAA AAAAAAAAAA
2551 AAAAAAAAA
**ANGABEN ZU SEQ ID NO: 6**
   SEQUENZCHARAKTERISTIKA:
      - LÄNGE:: 487 Aminosäuren
      - ART:: Aminosäuresequenz
   ART DES MOLEKÜLS: Protein
   URSPRÜNGLICHE HERKUNFT:
      - ORGANISMUS:: Homo Sapiens
      - STAMM:: kaukasisch
      - ENIWICKLUNGSSTADIUM:: adult
      - ZELLTYP:: epidermaler Keratinozyt
   UNMITTELBARE HERKUNFT: abgeleitet aus cDNA-Sequenz
   MERKMAL:
      - NAME/SCHLÜSSEL:: kodierende Sequenz für regulatorisches Protein pKe#83 aus humanen Keratinozyten .
      - LAGE:: von 1 bis 487
      - ERMITTLUNGSMETHODE:: abgeleitet aus cDNA-Sequenz
      - SONSTIGE ANGABEN: umfaßt eine Prenyl-Gruppen-Bindungsstelle ("CAAX-Box"), acht Proteinkinasephosphorylierungsmotive, 12 Caseinkinasephosphorylierungsmotive und zwei Tyrosinkinasephosphorylierungsmotive 
SEQ ID NO: 6
1 
51 
101 
151 
201 
251 
301 
351 
401 
451 *
**ANGABEN ZU SEQ ID NO 7:**
   SEQUENZCHARAKTERISTIKA:
      - LÄNGE:: 4911 Basenpaare
      - ART:: Desoxyribonukleinsäure
      - TOPOLOGIE:: linear
   ART DES MOLEKÜLS: cDNA
   HYPOTHETISCH: nein
   ANTI-SENSE: nein
   URSPRÜNGLICHE HERKUNFT:
      - ORGANISMUS:: Homo sapiens
      - STAMM:: kaukasisch
      - ENTWICKLUNGSSTADIUM:: adult
      - ZELLTYP:: epidermaler Keratinozyt
   UNMITTELBARE HERKUNFT: cDNA aus Keratinozyten
   MERKMAL:
      - NAME/SCHLÜSSEL:: kodierende Sequenz für regulatorisches Protein pKe#83 aus humanen Keratinozyten
      - LAGE:: von 1 bis 4911
      - ERMITTLUNGSMETHODE:: cDNA-Sequenzierung 
SEQ ID NO 7:

1 GGCGGGGGAG CCCTCCAGAA TACCCATCAT ATAGCCCCTG AGGTGGCATG
51 GTGATGTCTC CATGAGGGAA CCCCTTCCCA CTTCATACTG TCACGTATAT
101 CATAGTGTTC TTGACTGGGC CATTCATCTA AGATGGGATT TACCCTGTGA
151 AACAGGGAGA AGACTTATGG ACCCCAAGCA TCATTTCAAG TTGAAGTTGA
201 GTTTTTAAAA GCCATCCATG CAAAGTTCCT TTGCTTTGGA CCCTCTGCAT
251 TATTAAAGCT GCTGTATTGC TAACCCAGAA CTGCTCCAGT GTCTTGACTG
301 ATCATCATGG CTTCAGTTTG GAAGAGACTG CAGCGTGTGG GAAAACATGC
351 ATCCAAGTTC CAGTTTGTGG CCTCCTACCA GGAGCTCATG GTTGAGTGTA
401 CGAAGAAATG GTAACCAGAT AAACTGGTGG TAGTTTGGAC CAGAAGAAGC
451 CGAAGGAAGT CTTCTAAGGC ACATAGCTGG CAACCTGGAA TAAAAAATCC
501 CTATCGTGGT GTTGTTGTGT GGCCTGTTCC TGAAAACATT GAAATCACTG
551 TAACACTTTT TAAGGATCCT CATGCGGAAG AATTTGAAGA CAAAGAGTGG
601 ACATTTGTCA TAGAAAATGA ATCCCCTTCT GGTCGAAGGA AAGCTCTTGC
651 TACTAGCAGC ATCAATATGA AACAGTATGC AAGCCCTATG CCAACTCAGA
701 CTGATGTCAA GTTAAAATTC AAGCCATTAT CTAAAAAAGT TGTATCTGCC
751 GCTCTTCAGT TTTCATTATC TTGCATTTTT CTGAGGGAAG GAAAAGCCAC
801 AGATGAAGAC ATGCAAAGTT TGGCTAGTTT GGTGAGTATG AAGCAGGCTG
851 ACATTGGCAA TTTAGATGAC TTCGAAGAAG ATAATGAAGA TGATGATGAG
 
901 AACAGAGTGA ACCAAGAAGA AAAGGCAGCT AAAATTACAG AGCTTATCAA
951 CAAACTTAAC TTTTTGGATG AAGCAGAAAA GGACTTGGCC ACCGTGAATT
1001 CAAATCCATT TGATGATCCT GATGCTGCAG AATTAAATCC ATTTGGAGAT
1051 CCTGACTCAG AAGAACCTAT CACTGAAACA GCTTCACCTA GAAAAACAGA
1101 AGACTCTTTT TATAATAACA GCTATAATCC CTTTAAAGAG GTGCAGACTC
1151 CACAGTATTT GAACCCATTC GATGAGCCAG AAGCATTTGT GACCATAAAG
1201 GATTCTCCTC CCCAGTCTAC AAAAAGAAAA AATATAAGAC CTGTGGATAT
1251 GAGCAAGTAC CTCTATGCTG ATAGTTCTAA AACTGAAGAA GAAGAATTGG
1301 ATGAATCAAA TCCTTTTTAT GAACCTAAAT CAACTCCTCC TCCAAATAAT
1351 TTGGTAAATC CTGTTCAAGA ACTAGAAACT GAAAGGCGAG TGAAAAGAAA
1401 GGCCCCGGCT CCACCAGTCC TCTCACCAAA AACAGGAGTA TTAAATGAAA
1451 ACACAGTTTC TGCAGGAAAA GATCTCTCTA CTTCTCCTAA GCCAAGCCCT
1501 ATACCAAGTC CTGTTTTGGG GCGAAAGCCA AATGCTAGTC AGTCTTTGCT
1551 TGTATGGTGT AAAGAAGTTA CAAAGAACTA CCGAGGAGTA AAAATCACCA
1601 ATTTTACTAC ATCGTGGAGA AATGGTTTAT CTTTTTGTGC AATATTACAC
1651 CACTTTAGAC CAGATTTAAT TGACTACAAG TCTCTGAATC CTCAAGATAT
1701 TAAAGAGAAC AACAAAAAGG CATACGATGG ATTTGCCAGC ATAGGAATTT
1751 CCCGATTATT GGAACCTTCT GATATGGTAT TATTAGCAAT TCCTGATAAA
1801 CTGACTGTTA TGACTTATCT CTATCAAATA AGGGCACATT TCAGTGGCCA
1851 AGAACTAAAT GTCGTTCAGA TAGAGGAAAA CAGCAGTAAA AGCACATATA
1901 AAGTTGGAAA CTATGAAACA GATACAAACA GTTCTGTTGA TCAAGAAAAA
1951 TTCTATGCAG AGCTTAGTGA TCTGAAGCGG GAGCCTGAAC TACAACAGCC
2001 TATCAGCGGA GCAGTAGACT TCTTATCACA GGATGACTCT GTATTTGTAA
2051 ATGATAGCGG GGTTGGAGAG TCAGAAAGTG AGCATCAAAC TCCTGATGAT
2101 CACCTTAGTC CAAGCACAGC CTCCCCTTAC TGTCGCAGGA CTAAAAGTGA
2151 CACAGAACCC CAGAAGTCTC AGCAGAGCTC TGGAAGGACT TCAGGATCTG
2201 ATGACCCTGG AATATGTTCC AATACAGATT CAACCCAAGC ACAGGTTTTG
2251 TTAGGCAAAA AGAGACTATT GAAAGCTGAG ACTTTAGAAT TGAGTGACTT
2301 ATATGTTAGT GATAAGAAGA AGGATATGTC TCCACCCTTT ATTTGTGAGG
2351 AGACAGATGA ACAAAAGCTT CAAACTCTAG ACATCGGTAG TAACTTGGAG
2401 AAAGAAAAAT TAGAGAATTC CAGATCCTTA GAATGCAGAT CAGATCCAGA
2451 ATCTCCTATC AAAAAAACAA GTTTATCTCC TACTTCTAAA CTTGGATACT
2501 CATATAGTAG AGATCTAGAC CTTGCTAAGA AAAAACATGC TTCCCTGAGG
2551 CAGACGGAGT CTGATCCAGA TGCTGATAGA ACCACTTTAA ATCATGCAGA
2601 TCATTCATCA AAAATAGTCC AGCATCGATT GTTATCTAGA CAAGAAGAAC
2651 TTAAGGAAAG AGCAAGAGTT CTGCTTGAGC AAGCAAGAAG AGATGCAGCC
2701 TTAAAGGCGG GGAATAAGCA CAATACCAAC ACAGCCACCC CATTCTGCAA
2751 CAGGCAGCTA AGTGATCAGC AAGATGAAGA GCGACGTCGG CAGCTGAGAG
2801 AGAGAGCTCG TCAGCTAATA GCAGAAGCTC GATCTGGAGT GAAGATGTCA
2851 GAACTTCCCA GCTATGGTGA AATGGCTGCA GAAAAGTTGA AAGAAAGGTC
2901 AAAGGCATCT GGAGATGAAA ATGATAATAT TGAGATAGAT ACTAACGAGG
2951 AGATCCCTGA AGGCTTTGTT GTAGGAGGTG GAGATGAACT TACTAACTTA
3001 GAAAATGACC TTGATACTCC CGAACAAAAC AGTAAGTTGG TGGACTTGAA
3051 GCTGAAGAAG CTCCTAGAAG TTCAGCCACA GGTGGCAAAT TCACCCTCCA
3101 GTGCTGCCCA GAAAGCTGTA ACTGAGAGCT CAGAGCAGGA CATGAAAAGT
3151 GGCACAGAAG ATCTCCGGAC TGAACGATTA CAAAAAACAA CAGAACGTTT
3201 TAGAAATCCT GTTGTGTTCA GCAAAGATTC TACAGTCAGA AAAACTCAAC
3251 TTCAGTCTTT CAGCCAATAT ATTGAGAATA GACCAGAGAT GAAAAGGCAG
3301 AGATCAATAC AGGAAGATAC AAAGAAAGGA AATGAGGAGA AGGCAGCGAT
3351 AACTGAAACT CAGAGGAAGC CATCAGAAGA TGAAGTGCTT AATAAAGGGT
3401 TCAAAGACAC CAGTCAGTAT GTAGTAGGAG AATTGGCAGC ACTAGAGAAT
3451 GAGCAAAAGC AAATTGACAC CCGTGCCGCG CTGGTGGAGA AGCGCCTTCG
 
3501 CTATCTCATG GACACAGGAA GGAACACAGA AGAAGAAGAA GCTATGATGC
3551 AGGAATGGTT TATGTTAGTT AATAAGAAAA ATGCCTTAAT AAGGAGAATG
3601 AATCAGCTCT CTCTTCTGGA AAAAGAACAT GATTTAGAAC GACGGTATGA
3651 GCTGCTGAAC CGGGAATTGA GGGCAATGCT AGCCATTGAA GACTGGCAGA
3701 AGACCGAGGC CCAGAAGCGA CGCGAACAGC TTCTGCTAGA TGAGCTGGTG
3751 GCCCTGGTGA ACAAGCGCGA TGCGCTCGTC AGGGACCTGG ACGCGCAGGA
3801 GAAGCAGGCC GAAGAAGAAG ATGAGCATTT GGAGCGAACT CTGGAGCAAA
3851 ACAAAGGCAA GATGGCCAAG AAAGAGGAGA AATGTGTTCT TCAGTAGCCA
3901 TCAGATCAGA AAGAATCTCT CCCAACATTT TAGAGTCTTG CTTCCCAAAC
3951 CAGAAAAAGT CAGACTCATT GTTGATTTAA AACTTTTAAC ATTTTGTTTG
4001 GCTGGATTGT ACTACTTTAC CTCTACTTTA CCACCACCAC CCTTTTCCTC
4051 CCTCCTTTCC AAATAATATA CAGAACTCCA AAATAGCTTC ATTTAAGGAT
4101 TTTTTTGTGA GTTAACAATT TCCTTGAAAT CCTGTGAAAT AGATTTGCAC
4151 AGACACCTTG TGAGTGATTG GTATTGGAGG TGTTCAAGAA ACTGTTCGAA
4201 AAAGAACAAA AACACTTCCC TCGTTATTTT CTCTCATTTT TTGATGAGAG
4251 GAAAATTTGA AACATTATTC TTGTTGTTGT TGGTAATAGC ATAATGACAG
4301 TGGGAGGGGT ACAAGGGGAT AAGAAAAATG TCATGATTTT TTTCCGGTCC
4351 TGCCACATGT AACACTTACT CTGTTACCTA AATTTTATAG TTAGATCATA
4401 TCCAATCTAC TTATTAAACT GTGTTCTATT TACCAGTGGA GTTTTTCTGC
4451 AGTGGTTGCG TTTCACTGTA AGGATAATGG AGTTCCTCTC CTCTGCTTTC
4501 CTCAGAGGAT GGTCCTTTAA CATAGCCAGA AACAAGCCCT GTGGTTTGAA
4551 GGTGAGCTGT GAGGATGGGA CTAATTGATA TGCACCAGTT TACAAAGACA
4601 GTCTTATCAT CCGAGAATAC ACCATCTTTT TCTCTGGATA ATTATTTCTT
4651 ACATCATGCT TGATTCCTAC ATTTTGTTGG GTTTCAACAT TGGCTCACGA
4701 ATGCTGTTAA TATTTATTCT GTATTGATAA AAAGTCTGTC TTGCCACTAC
4751 AAGTAAATCC CCCATTTAAT ATTTTCTTCT TTAGCATAGC ACTGTCATTT
4801 TTTGTGAAAA TGGTTATGTT TATTTATTAC AATACTGAGT CATATATAAA
4851 TTTTCAATAA AAGCAGAAAC TTTCTTACCT TAAAAAAAAA AAAAAAAAAA
4901 AAAAAAAAAA A*

(END)
**ANGABEN ZU SEQ ID-NO 8:**
   SEQUENZCHARAKTERISTIKA:
      - LÄNGE:: 1076 Aminosäuren
      - ART:: Aminosäuresequenz
   ART DES MOLEKÜLS: Protein
   URSPRÜNGLICHE HERKUNFT:
      - ORGANISMUS:: Homo sapiens
      - STAMM:: kaukasisch
      - ENTWICKLUNGSSTADIUM:: adult
      - ZELLTYP:: epidermaler Keratinozyt
   UNMITTELBARE HERKUNFT: abgeleitet aus cDNA-Sequenz
   MERKMAL:
      - NAME/SCHLÜSSEL:: kodierende Sequenz für das regulatorische Protein pKe#83 aus humanen Keratinozyten
      - LAGE:: von 1 bis 1076
      - ERMITTLUNGSMETHODE:: Ableitung aus cDNA-Sequenz
      - SONSTIGE ANGABEN:: umfaßt eine Prenyl-Gruppen-Bindungsstelle ("CAAX-Box"), 24 Proteinkinasephosphorylierungsmotive, 29 Caseinkinasephosphorylierungsmotive 5 Tyrosinkinasephosphorylierungsmotive und 8 Myrsitylierungsstellen

SEQ ID-NO. 8:

1 MKQYASPMPT QTDVKLKFKP LSKKVVSAAL QFSLSCIFLR EGKATDEDMQ
51 SLASLVSMKQ ADIGNLDDFE EDNEDDDENR VNQEEKAAKI TELINKLNFL
101 DEAEKDLATV NSNPFDDPDA AELNPFGDPD SEEPITETAS PRKTEDSFYN
151 NSYNPFKEVQ TPQYLNPFDE PEAFVTIKDS PPQSTKRKNI RPVDMSKYLY
201 ADSSKTEEEE LDESNPFYEP KSTPPPNNLV NPVQELETER RVKRKAPAPP
251 VLSPKTGVLN ENTVSAGKDL STSPKPSPIP SPVLGRKPNA SQSLLVWCKE
301 VTKNYRGVKI TNFTTSWRNG LSFCAILHHF RPDLIDYKSL NPQDIKENNK
351 KAYDGFASIG ISRLLEPSDM VLLAIPDKLT VMTYLYQIRA HFSGQELNVV
401 QIEENSSKST YKVGNYETDT NSSVDQEKFY AELSDLKREP ELQQPISGAV
451 DFLSQDDSVF VNDSGVGESE SEHQTPDDHL SPSTASPYCR RTKSDTEPQK
501 SQQSSGRTSG SDDPGICSNT DSTQAQVLLG KKRLLKAETL ELSDLYVSDK
551 KKDMSPPFIC EETDEQKLQT LDIGSNLEKE KLENSRSLEC RSDPESPIKK
601 TSLSPTSKLG YSYSRDLDLA KKKHASLRQT ESDPDADRTT LNHADHSSKI
651 VQHRLLSRQE ELKERARVLL EQARRDAALK AGNKHNTNTA TPFCNRQLSD
701 QQDEERRRQL RERARQLIAE ARSGVKMSEL PSYGEMAAEK LKERSKASGD
751 ENDNIEIDTN EEIPEGFVVG GGDELTNLEN DLDTPEQNSK LVDLKLKKLL
801 EVQPQVANSP SSAAQKAVTE SSEQDMKSGT EDLRTERLQK TTERFRNPVV
851 FSKDSTVRKT QLQSFSQYIE NRPEMKRQRS IQEDTKKGNE EKAAITETQR
901 KPSEDEVLNK GFKDTSQYVV GELAALENEQ KQIDTRAALV EKRLRYLMDT
951 GRNTEEEEAM MQEWFMLVNK KNALIRRMNQ LSLLEKEHDL ERRYELLNRE
1001 LRAMLAIEDW QKTEAQKRRE QLLLDELVAL VNKRDALVRD LDAQEKQAEE
1051 EDEHLERTLE QNKGKMAKKE EKCVLQ*

## Patentansprüche

1. Isoliertes Polypeptid,
das einem natürlicherweise in humanen epidermalen Keratinozyten vorkommenden und im aktivierten, durch eine erhöhte Expression der Aktivierungsmarker uPA und uPA-R gekennzeichneten Zustand dieser Keratinozyten aufregulierten, nämlich verstärkt exprimierten Protein in Funktion und Wirkung gleicht ,
und das entweder die im Sequenzprotokoll SEQ ID NO: 3 oder SEQ ID NO: 4 oder SEQ ID NO: 6 dargestellte oder eine durch Aminosäuresubstitution, -deletion, -insertion, -oder -inversion als Allel oder Derivat daraus entstandene Aminosäuresequenz aufweist,
wobei die in den Sequenzprotokollen dargestellten und die durch Aminosäuresubstitution, -deletion, -insertion, -oder -inversion als Allel oder Derivat daraus entstandenen Aminosäuresequenzen zur Beeinflussung der Zellmorphologie, Zellproliferation, Zelladhäsion, Zellmigration und/oder Zelldifferenzierung geeignet sind.

2. Isolierte Nukleinsäure,
die ein Protein gemäß Anspruch 1 codiert,
und die entweder die im Sequenzprotokoll SEQ ID NO: 1 dargestellte Nukleotidsequenz oder eine hierzu komplementäre Nukleotidsequenz aufweist.

3. Isolierte Nukleinsäure nach Anspruch 2, **dadurch gekennzeichnet,**
**daß** diese Nukleinsäure aus einer natürlichen, synthetischen oder halbsynthetischen Quelle gewonnen ist.

4. Isolierte Nukleinsäure nach Anspruch 2 oder 3 **dadurch gekennzeichnet,**
**daß** diese Nukleinsäure eine cDNA ist.

5. Verwendung einer isolierten Nukleinsäure nach einem der Ansprüche 2 oder 3 zur Herstellung eines Sense- oder Antisense-Oligonukleotids, das mindestens 6, vorzugsweise 8 bis 25 Nukleotide umfaßt und mit der im Sequenzprotokoll SEQ ID NO: 1 oder mit der im Sequenzprotokoll SEQ ID NO: 7 dargestellten Nukleotidsequenz unter stringenten Hybridisierungsbedingungen hybridisiert.

6. Verwendung einer isolierten Nukleinsäure nach einem der Ansprüche 2 oder 3 zur Herstellung einer Splice-Variante, die mit der im Sequenzprotokoll SEQ ID NO: 1 oder mit der im Sequenzprotokoll SEQ ID NO: 7 dargestellten Nukleotidesquenz unter stringenten Hybridisierungsbedingungen hybridisiert.

7. Verwendung nach Anspruch 6, **dadurch gekennzeichnet,**
**daß** die Splice-Variante die im Sequenzprotokoll SEQ ID NO: 2 oder SEQ ID NO: 5 dargestellte Nukleotidsequenz aufweist.

8. Isoliertes Polypeptid , **dadurch gekennzeichnet,**
**daß** es eine Aminosäuresequenz aufweist, die aus einer Splice-Variante einer mRNA resultiert, welche
entweder die im Sequenzprotokoll SEQ ID NO: 1 dargestellte Nukleotidsequenz oder eine hierzu komplementäre Nukleotidsequenz oder eine Teilsequenz einer dieser Nukleotidsequenzen
oder eine ganz oder teilweise mit einer dieser Nukleotidsequenzen unter stringenten Hybridisierungsbedingungen hybridisierende Nukleotidsequenz aufweist,
**daß** es in aktivierten humanen epidermalen Keratinozyten mit erhöhter Expression der Aktivierungsmarker uPA und uPA-R aufreguliert wird,
und **daß** es zur Beeinflussung der Zellmorphologie, Zellproliferation, Zelladhäsion, Zellmigration und/oder Zelldifferenzierung geeignet ist.

9. Isoliertes Polypeptid, **dadurch gekennzeichnet,**
**daß** es eine Aminosäuresequenz aufweist, die aus einer Splice-Variante einer mRNA resultiert, welche die im Sequenzprotokoll SEQ ID NO: 2 oder im Sequenzprotokoll SEQ ID NO: 5 dargestellte Nukleotidsequenz aufweist.

10. Isoliertes Polypeptid nach Anspruch 9, **dadurch gekennzeichnet,**
**daß** es die im Sequenzprotokoll SEQ ID NO: 4 oder die im Sequenzprotokoll SEQ ID NO: 6 dargestellte Aminosäuresequenz aufweist.

11. Rekombinantes DNS-Vektormolekül, das eine Nukleinsäure nach einem der Ansprüche 2 bis 4 oder eine nach Anspruch 6 oder 7 hergestellte Splice-Variante der Nukleotidsequenz gemäß Sequenzprotokoll **SEQ ID NO: 1** umfaßt, und das die Fähigkeit zur Expression eines in humanen Keratinozyten vorkommenden und in aktivierten Keratinozyten verstärkt exprimierten Proteins in einer prokaryontischen oder eukaryontischen Zelle aufweist.

12. Rekombinantes DNS-Vektormolekül nach Anspruch 11, **dadurch gekennzeichnet, daß** das Vektormolekül ein Abkömmling des Plasmids pUEX-1 oder des Plasmids pGEX-2T oder des Plasmids pcDNA3.1 ist.

13. Rekombinantes DNS-Vektormolekül nach Anspruch 12, **dadurch gekennzeichnet, daß** das Vektormolekül ein Konstrukt gemäß Fig. 2 oder gemäß Fig. 3 ist.

14. Transformierte Wirtszelle, die eine Nukleinsäure nach einem der Ansprüche 2 bis 4 oder eine nach Anspruch 6 oder 7 hergestellte Splice-Variante der Nukleotidsequenz gemäß Sequenzprotokoll **SEQ ID NO: 1** enthält, welche mit einem aktivierbaren Promotor gekoppelt ist, der in der Wirtszelle natürlicherweise oder als Folgen einer Rekombination enthalten ist, und die die Fähigkeit zur Expression eines in humanen Keratinozyten vorkommenden und in aktivierten Keratinozyten verstärkt exprimierten Proteins aufweist.

15. Transformierte Wirtszelle nach Anspruch 14, **dadurch gekennzeichnet, daß** der Promotor der Zytokeratin-14-Promotor und die Wirtszelle ein Keratinozyt ist, oder daß der Promotor der CMV-Promotor und die Wirtszelle eine Cos-Zelle ist.

16. Verwendung einer Nukleinsäure nach Anspruch 2 oder eines Vektormoleküls nach einem der Ansprüche 11 bis 13 zur Herstellung transgener Säugetiere, ausgenommen Menschen.

17. Verwendung eines Polypeptids nach Anspruch 1 oder Anspruch 8 zur Herstellung eines Antikörpers gegen dieses Polypeptid.

18. Verwendung nach Anspruch 17, **dadurch gekennzeichnet, daß** der Antikörper zur diagnostischen und/oder therapeutischen Behandlung von dermatologischen Erkrankungen oder zur kosmetischen Behandlung eingesetzt wird.

19. Antikörper, der spezifisch mit einem Polypeptid gemäß Anspruch 1 oder Anspruch 8 reagiert.

20. Reagenz zum indirekten Nachweis eines in humanen Keratinozyten vorkommenden und in aktivierten Keratinozyten verstärkt exprimierten Proteins, **dadurch gekennzeichnet, daß** das Reagenz unter Verwendung wenigstens einer Nukleinsäure nach einem der Ansprüche 2 bis 4 oder einer nach Anspruch 6 oder 7 hergestellten Splice-Variante der Nukieotidsequenz gemäß Sequenzprotokoll **SEQ ID NO: 1** und/oder eines Polypeptids gemäß Anspruch 1 oder Anspruch 8 hergestellt ist.

21. Verwendung nach Anspruch 5 oder Anspruch 6 zur Herstellung eines Mittels für die diagnostische und/oder therapeutische Behandlung von dermatologischen Erkrankungen oder zur kosmetischen Behandlung.

22. Verwendung eines Polypeptids nach Anspruch 1 oder Anspruch 8 oder einer Nukleinsäure nach Anspruch 2 zur Identifizierung von medizinisch, kosmetisch oder pharmakologisch einsetzbaren Substanzen, die an das Polypeptid bzw. die Nukleinsäure binden und dadurch dessen/deren Funktion und/oder Expression beeinflussen.

## Claims

1. Isolated polypeptide,
which is identical in its function and effect to a protein that occurs naturally in human epidermal keratinocytes and is upwardly adjusted, specifically increasingly expressed when the keratinocytes are in an activated state **characterized by** an elevated expression of the activation markers uPA and uPA-R, and
which has the amino acid sequence indicated in either the SEQ ID NO:3 or SEQ ID NO:4 or SEQ ID NO:6 sequence protocol, or an allele or derivative obtained through amino acid substitution, deletion, insertion or inversion from the latter,
and wherein the amino acid sequences obtained through amino acid substitution, deletion, insertion or inversion as an allele or derivative are suitable for influencing cell morphology, cell proliferation, cell adhesion, cell migration and/or cell differentiation.

2. Isolated nucleic acid,
that codes a protein according to claim 1, and that has the nucleotide sequence indicated in the SEQ ID NO:1 sequence protocol or a nucleotide sequence complementary to this

3. Isolated nucleic acid according to claim 2, **characterized by** the fact that this nucleic acid is obtained from a natural, synthetic or half-synthetic source.

4. Isolated nucleic acid according to claim 2 or 3, **characterized by** the fact that this nucleic acid is a cDNA.

5. Use of an isolated nucleic acid according to one of claims 2 or 3 for manufacturing a sense or antisense oligonucleotide, which encompasses at least 6, preferably 8 to 25 nucleotides, and hybridizes under stringent hybridization conditions with the nucleotide sequence indicated in sequence protocol SEQ ID NO:1 or sequence protocol SEQ ID NO:7.

6. Use of an isolated nucleic acid according to one of claims 2 or 3 for manufacturing a splice variant, which hybridizes under stringent hybridization conditions with the nucleotide sequence indicated in sequence protocol SEQ ID NO:1 or in sequence protocol SEQ ID NO:7.

7. Use according to claim 6, **characterized by** the fact that the splice variant has the nucleotide sequence indicated in sequence protocol SEQ ID NO: 2 or SEQ ID NO: 5.

8. Isolated polypeptide, **characterized in that** it has an amino acid sequence resulting from a splice variant of an mRNA, which
has either the nucleotide sequence indicated in sequence protocol SEQ ID NO:1, or a nucleotide sequence complementary to this, or a partial sequence of one of these nucleotide sequences,
or a nucleotide sequence that hybridizes under stringent hybridization conditions wholly or in part with one of these nucleotide sequences,
that it is upwardly adjusted in activated human epidermal keratinocytes showing an elevated expression of the activation markers uPA and uPA-R,
and that it is suitable for influencing cell morphology, cell proliferation, cell adhesion, cell migration and/or cell differentiation.

9. Isolated polypeptide, **characterized by** the fact that it has an amino acid sequence resulting from a splice variant of an mRNA, which has the nucleotide sequence indicated in sequence protocol SEQ ID NO:2 or sequence protocol SEQ ID NO:5.

10. Isolated polypeptide according to claim 9, **characterized by** the fact that it has the amino acid sequence indicated in sequence protocol SEQ ID NO:4 or sequence protocol SEQ ID NO:6.

11. Recombinant DNS vector molecule, which encompasses a nucleic acid according to one of claims 2 to 4, or a splice variant of the nucleotide sequence according to sequence protocol SEQ ID NO:1 that is manufactured according to claim 6 or 7, and which has the ability to express a protein that occurs in human keratinocytes and is increasingly expressed in activated keratinocytes, in a prokaryotic or eukaryotic cell.

12. Recombinant DNS vector molecule according to claim 11, **characterized by** the fact that the vector molecule is a derivative of the plasmid pUEX-1 or plasmid pGEX-2T or plasmid pcDNA3.1.

13. Recombinant DNS vector molecule according to claim 12, **characterized by** the fact that the vector molecule is a construct according to the vector protocol on Fig. 2 or the vector protocol on Fig. 3.

14. Transformed host cell containing a nucleic acid according to one of claims 2 to 4, or a splice variant of the nucleotide sequence according to sequence protocol SEQ ID NO:1 which is manufactured according to claim 6 or 7, wherein said nucleic acid or splice variant is coupled with an activatable promotor contained in the host cell naturally or as the consequence of a recombination, and has the ability to express a protein that occurs in human keratinocytes and that is increasingly expressed in activated keratinocytes.

15. Transformed host cell according to claim 14, **characterized by** the fact that the promotor is the cytokeratin-14 promotor and the host cell is a keratinocyte, or that the promotor is the CMV promotor and the host cell is a cos cell.

16. Use of a nucleic acid according to claim 2 or a vector molecule according to one of claims 11 to 13 for manufacturing transgenic mammals -except humans.

17. Use of a polypeptide according to claim 1 or claim 8 for manufacturing an antibody against this polypeptide.

18. Use according to claim 17, **characterized by** the fact that the antibody is used for the diagnostic and/or therapeutic treatment of dermatological diseases, or for the cosmetic treatment.

19. Antibody that reacts specifically with a polypeptide according to claim 1 or claim 8.

20. Reagent for the indirect detection of a protein that occurs in human keratinocytes and is increasingly expressed in activated keratinocytes, **characterized by** the fact that the reagent is manufactured using at least one nucleic acid according to one of claims 2 to 4, or a splice variant of the nucleotide sequence according to sequence protocol SEQ ID NO:1 that is manufactured according to claim 6 or 7, and/or a polypeptide according to claim 1 or claim 8.

21. Use according to claim 5 or claim 6 for manufacturing a means for the diagnostic and/or therapeutic treatment of dermatological diseases, or for cosmetic treatment.

22. Use of a polypeptide according to claim 1 or claim 8 or a nucleic acid according to claim 2 for identifying substances suitable for medical, cosmetic or pharmacological applications, which bind to the polypeptide or nucleic acid, and thereby influence its function and/or expression.

## Revendications

1. Polypeptide isolé
qui est semblable en fonction et en action à une protéine exprimé de façon accrue apparaissant naturellement dans des kératinocytes épidermiques humains et qui dans un état activé, **caractérisé par** une expression plus élevée du marqueur d'activation uPA et uPA-R, régule ce kératinocyte,
et qui présente la séquence d'acides aminés représentée dans le protocole de séquences SEQ ID N0 : 3 ou SEQ ID NO : 4 ou SEQ ID NO : 6 ou une séquence d'acides aminés obtenue sous forme d'allèle ou de dérivé de celles-ci par substitution, délétion insertion ou inversion d'acides aminés,
les séquences d'acides aminés représentées dans le protocole de séquences et les séquences d'acides aminés obtenues à partir de celles-ci sous forme d'allèle ou de dérivé par substitution, délétion, insertion ou inversion d'acides aminés étant aptes à influencer la morphologie de la cellule, la prolifération de la cellule, l'adhésion de la cellule, la migration de la cellule et/ou la différentiation de la cellule.

2. Acide nucléique isolé qui code une protéine selon la revendication 1 et qui présente soit la séquence de nucléotide représentée dans le protocole de séquences SEQ ID NO : 1 ou une séquence de nucléotides complémentaire de celle-ci.

3. Acide nucléique isolé selon la revendication 2, **caractérisé en ce que** celui-ci est obtenu à partir d'une source naturelle, synthétique ou semi-synthétique.

4. Acide nucléique isolé selon la revendication 2 ou 3, **caractérisé en ce que** cet acide nucléique est un cDNA.

5. Utilisation d'un acide nucléique isolé selon l'une des revendications 2 ou 3 pour la fabrication d'un oligonucléotide sens ou anti-sens qui comprend au moins 6, de préférence entre 8 et 25, nucléotides et qui s'hybride avec la séquence de nucléotides représentée dans le protocole de séquences SEQ ID NO : 1 ou avec la séquence de nucléotides représentée dans le protocole de séquences SEQ ID NO: 7 dans des conditions d'hybridation strictes.

6. Utilisation d'un acide nucléique isolé selon la revendication 2 ou 3 pour la fabrication d'une variante d'épissage qui s'hybride avec la séquence de nucléotides représentée dans le protocole de séquence SEQ ID NO: 1 ou avec la séquence de nucléotides représentée dans le protocole de séquences SEQ ID NO : 7 dans des conditions d'hybridation strictes.

7. Utilisation selon la revendication 6, **caractérisée en ce que** la variante d'épissage présente la séquence de nucléotides représentée dans le protocole de séquences SEQ ID NO : 2 ou SEQ ID NO : 5.

8. Polypeptide isolé, **caractérisé en ce que**
il présente une séquence d'acides aminés qui résulte d'une variante d'épissage d'un mARN, qui
présente soit la séquence de nucléotides représentée au protocole de séquences SEQ ID NO: 1 ou une séquence de nucléotides complémentaire de celle-ci ou une séquence partielle d'une de ces séquences de nucléotides
soit une séquence de nucléotides partiellement ou totalement hybridée avec une de ces séquences de nucléotides dans des conditions d'hybridation strictes
**en ce qu'**il est régulé dans des kératinocytes épidermiques humains activés avec une expression accrue du marqueur d'activation uPA et uPA-R,
et **en ce qu'**il est apte à influencer la morphologie de la cellule, la prolifération de la cellule, l'adhésion de la cellule, la migration de la cellule et/ou la différentiation de la cellule.

9. Polypeptide isolé **caractérisé en ce qu'**il présente une séquence d'acides aminés qui résulte d'une variante d'épissage d'un mARN qui présente la séquence de nucléotides représentée dans le protocole de séquences SEQ ID NO: 2 ou celle représentée dans le protocole de séquences SEQ ID NO : 5.

10. Polypeptide isolé selon la revendication 9, **caractérisé en ce qu'**il présente la séquence d'acides aminés représentée dans le protocole de séquences SEQ ID NO : 4 ou celle représentée dans le protocole de séquences SEQ ID NO : 6.

11. Molécule vectrice d'ADN recombinant qui comprend un acide nucléique selon l'une des revendications 2 à 4 ou une variante d'épissage de la séquence de nucléotides selon le protocole de séquence SEQ ID NO : 1 réalisée selon la revendication 6 ou 7, et qui est apte à exprimer dans une cellule procaryote ou eucaryote une protéine apparaissant dans des kératinocytes humains et exprimée de façon accrue dans des kératinocytes activés.

12. Molécule vectrice d'ADN recombinant selon la revendication 11, **caractérisée en ce que** la molécule vectrice est une descendance du plasmide pUEX-1 ou du plasmide pGEX-2T ou du plasmide pcDNA3.1.

13. Molécule vectrice d'ADN recombinant selon la revendication 12, **caractérisée en ce que** la molécule vectrice est une création selon la figure 2 ou selon la figure 3.

14. Cellule hôte transformée qui contient un acide nucléique selon l'une des revendications 2 à 4 ou une variante d'épissage de la séquence de nucléotides selon le protocole de séquences SEQ ID NO : 1 réalisée selon la revendication 6 ou 7, qui est couplée à un promoteur activable contenu dans la cellule hôte naturellement ou suite à une recombinaison, et qui est apte à exprimer une protéine apparaissant dans des kératinocytes humains et exprimée de façon accrue dans les kératinocytes activés.

15. Cellule hôte transformée selon la revendication 14, **caractérisée en ce que** le promoteur est le promoteur de zytokératine 14 et la cellule hôte est un kératinocyte, ou que le promoteur est le promoteur de CMV et la cellule hôte une cellule Cos.

16. Utilisation d'un acide nucléique selon la revendication 2 ou d'une molécule vectrice selon l'une des revendications 11 à 13 pour la fabrication de mammifères transgéniques, à l'exception de l'homme.

17. Utilisation d'un polypeptide selon la revendication 1 ou la revendication 8 pour la fabrication d'un anticorps contre ce polypeptide.

18. Utilisation selon la revendication 17, **caractérisée en ce que** l'anticorps est utilisé pour un traitement diagnostique et/ou thérapeutique de maladies dermatologiques ou pour le traitement cosmétiques.

19. Anticorps qui réagit de façon spécifique avec un polypeptide selon la revendication 1 ou la revendication 8.

20. Réactif pour la détection indirecte d'une protéine apparaissant dans des kératinocytes humains et exprimée de façon accrue dans des kératinocytes activés, **caractérisé en ce que** le réactif est fabriqué en utilisant au moins un acide nucléique selon l'une des revendications 2 à 4 ou une variante d'épissage de la séquence de nucléotides selon le protocole de séquences SEQ ID NO: 1 réalisée selon la revendication 6 ou 7 et/ou un polypeptide selon la revendication 1 ou 8.

21. Utilisation selon la revendication 5 ou la revendication 6 pour la fabrication d'un produit pour le traitement diagnostique et/ou thérapeutique de maladies dermatologiques ou pour le traitement cosmétique.

22. Utilisation d'un polypeptide selon la revendication 1 ou la revendication 8 ou d'un acide nucléique selon la revendication 2 pour identifier de substances utilisables en médecine, en cosmétique ou en pharmacologie qui se lient au polypeptide ou à l'acide nucléique et ainsi influencent sa fonction et/ou son expression.
